# EUROPEAN PATENT APPLICATION

(11) **EP 4 523 660 A1**
(43) Date of publication of application: **19.03.2025**
(21) Application number: 24200404.2
(22) Date of filing: 13.09.2024
(51) Int. Cl.: A61F 2/28, A61F 2/30

(54) **HUMERAL LIMB SALVAGE IMPLANT SYSTEM**

(30) Priority: 15.09.2023 US 202363538658 P
(71) Applicant: Zimmer, Inc., Warsaw, IN 46580 (US)
(72) Inventor: DYE, Donald W., Warsaw, 46582 (US)
(74) Representative: Taor, Simon Edward William

(57) **Abstract**

A limb replacement implant (100, 300,500,600,700) including a shaft (122,132,142,152,302,502,608) configured to simulate an anatomical shape of a natural humerus, the shaft extending from a proximal end to a distal end. The limb replacement implant including a patch (208, 228, 248, 268, 288, 210, 230, 250, 270, 304, 306, 508, 510, 620, 610) positioned on a portion of the shaft. The patch is at least one of: positioned, sized or shaped as derived from an anatomical counterpart attachment from the natural humerus.

## Description

### BACKGROUND

The humerus bone is located in the upper arm, and forms joints at the shoulder and the elbow. A variety of soft tissue including muscles and ligaments attach to the humerus along the elongate length of the humerus. Portions of the humerus may need to be replaced due to disease, injury, and/or a need to replace portions of the shoulder joint or elbow joint.

### OVERVIEW

Humeral replacement limbs have traditionally been fabricated from bar stock. Bar stock is generally heavy. Such bar stock formed implants typically have suture holes that are machined or added during surgery with screws for anchoring purposes. Bar stock implants can also have additional parts added. Because the implants use round bar stock as a base material, round rods are the basis shape for the implant designs and lead to modular implants that are not shaped like a natural humerus.

The present inventor has recognized with the present disclosure a simplified humeral replacement limb design that can be anatomically shaped to better simulate a natural limb. Additionally, the present inventor's designs can preserve existing natural bone as further discussed herein. Such a bone saving design can have a positive impact on clinical outcomes.

As an example, the humerus replacement implant (sometimes simply referred to as "implant" or "implants" herein for brevity) can be made using additive manufacturing technology. Using the techniques described here, a humerus replacement implant can be made that is substantially similar in size and shape to a humerus bone. The humerus replacement implant can create a flush or smooth implant that cannot be achieved without significant time and/or cost using bar stock. Additionally, the humerus replacement implants of the present application are designed to eliminate the need to "bolt on" or other modular addition of parts to the implant, thereby maintaining a smooth contour of the implant. By maintaining a smooth contour, musculature and other soft tissue can more easily interface with the humeral implant in order to function. In addition, the smooth contour can reduce impingement or irritation of surrounding soft tissue, thereby reducing patient discomfort.

The humerus replacement implant of the present disclosure can also include one or more integral, flush, and/or contoured patches that are substantially similar to the porous structure of a human cancellous bone. In addition, the humeral implant can include one or more integral attachment features to receive one or more sutures. The implant can include a plurality of integral attachment features, which can surround the one or more patches. Additionally, or alternatively, the integral attachment features can be positioned within the patches. The patches and/or integral attachment features can be positioned on the implant to align to zones specific to anatomic muscle attachment areas. Providing a large number of integral attachment features and patches can simplify the surgery. For example, there is no need to bolt on separate parts that can cause roughened surfaces and the creation of suture holes. Instead, the integral attachment features are readily available and accessible. In addition, the patches are positioned in anatomically correct locations and flush with the implant external contours.

The porous structure such as those utilized at the patches discussed herein can be formed at least partially of a porous material. The porous material can have a substrate that incorporates one or more of a variety of biocompatible metals such as aluminum, titanium, a titanium alloy, cobalt chromium, cobalt chromium molybdenum, tantalum, or a tantalum alloy. According to an exemplary embodiment of the present disclosure, the substrate may be a Ti-6Al-4V alloy, such as Tivanium^{®}, OsseoTi^{®} or other porous which is available from Zimmer Biomet, Inc., of Warsaw, Ind. The porous material can have a highly porous metal structure that can incorporate one or more of a variety of biocompatible metals. Such structures are particularly suited for contacting bone and soft tissue, and in this regard, can be useful as a bone substitute and as cell and tissue receptive material, for example, by allowing tissue to grow into the porous structure over time to enhance fixation (i.e., osseointegration) between the structure and surrounding bodily structures. According to certain embodiments of the present disclosure, an open porous metal structure may have a porosity as low as 55%, 65%, or 75% or as high as 80%, 85%, or 90%, or within any range defined between any pair of the foregoing values. An example of an open porous metal structure is produced using Trabecular Metal^{™} Technology available from Zimmer Biomet, Inc. Such a material may be formed from a reticulated vitreous carbon foam substrate which is infiltrated and coated with a biocompatible metal, such as tantalum, by a chemical vapor deposition ("CVD") process in the manner disclosed in detail in U.S. Patent No. 5,282,861 and in Levine, B.R., et al., "Experimental and Clinical Performance of Porous Tantalum in Orthopedic Surgery", Biomaterials 27 (2006) 4671-4681, the disclosures of which are expressly incorporated herein by reference. In addition to tantalum, other biocompatible metals may also be used in the formation of a highly porous metal structure such as aluminum, titanium, a titanium alloy, cobalt chromium, cobalt chromium molybdenum, tantalum, a tantalum alloy, niobium, or alloys of tantalum and niobium with one another or with other metals. It is also within the scope of the present disclosure for a porous metal structure to be in the form of a fiber metal pad or a sintered metal layer, such as a Cancellous-Structured Titanium^{™} (CSTi^{™}) layer. CSTi^{™} porous layers are manufactured by Zimmer Biomet, Inc.

The highly porous metal structure will include a large plurality of metallic ligaments defining open voids (i.e., pores) or channels therebetween. The open spaces between the ligaments form a matrix of continuous channels having few or no dead ends, such that growth of soft tissue and / or bone through open porous metal is substantially uninhibited. Thus, the open porous metal may provide a lightweight, strong porous structure which is substantially uniform and consistent in composition, and provides a matrix (e.g., closely resembling the structure of natural cancellous bone) into which soft tissue and bone may grow to provide fixation of the implant to surrounding bodily structures. According to some aspects of the present disclosure, exterior surfaces of an open porous metal structure can feature terminating ends of the above-described ligaments. Such terminating ends can be referred to as struts, and they can generate a high coefficient of friction along an exposed porous metal surface. Such features can impart an enhanced affixation ability to an exposed porous metal surface for adhering to bone and soft tissue. Also, when such highly porous metal structures are coupled to an underlying substrate, a small percentage of the substrate may be in direct contact with the ligaments of the highly porous structure, for example, approximately 15%, 20%, or 25%, of the surface area of the substrate may be in direct contact with the ligaments of the highly porous structure.

An open porous metal structure may also be fabricated such that it comprises a variety of densities in order to selectively tailor the structure for particular orthopedic applications. In particular, as discussed in the above-incorporated U.S. Patent No. 5,282,861, an open porous metal structure may be fabricated to virtually any desired density, porosity, and pore size (e.g., pore diameter), and can thus be matched with the surrounding natural tissue in order to provide an improved matrix for facilitating tissue ingrowth and mineralization. According to certain embodiments, an open porous metal structure may be fabricated to have a substantially uniform porosity, density, and/or void (pore) size throughout, or to comprise at least one of pore size, porosity, and/or density being varied within the structure. For example, an open porous metal structure may have a different pore size and/or porosity at different regions, layers, and surfaces of the structure. The ability to selectively tailor the structural properties of the open porous metal, for example, enables tailoring of the structure for distributing stress loads throughout the surrounding tissue and promoting specific tissue ingrown within the open porous metal.

An open porous metal structure may comprise an open cell polyurethane foam substrate coated with Ti-6Al-4V alloy using a low temperature arc vapor deposition process. Ti-6Al-4V beads may then be sintered to the surface of the Ti-6Al-4V-coated polyurethane foam substrate. Additionally, another embodiment of an open porous metal structure may comprise a metal substrate combined with a Ti-6Al-4V powder and a ceramic material, which is sintered under heat and pressure. The ceramic particles may thereafter be removed leaving voids, or pores, in the substrate. An open porous metal structure may also comprise a Ti-6Al-4V powder which has been suspended in a liquid and infiltrated and coated on the surface of a polyurethane substrate. The Ti-6Al-4V coating may then be sintered to form a porous metal structure mimicking the polyurethane foam substrate. Further, another embodiment of an open porous metal structure may comprise a porous metal substrate having particles, comprising altered geometries, which are sintered to a plurality of outer layers of the metal substrate. Additionally, an open porous metal structure may be fabricated according to electron beam melting (EBM) and/or laser engineered net shaping (LENS). For example, with EBM, metallic layers (comprising one or more of the biomaterials, alloys, and substrates disclosed herein) may be coated (layer by layer) on an open cell substrate using an electron beam in a vacuum. Similarly, with LENS, metallic powder (such as a titanium powder, for example) may be deposited and coated on an open cell substrate by creating a molten pool (from a metallic powder) using a focused, high-powered laser beam.

The humerus replacement implant may have a hollow implant structure. The hollow structure may substantially reduce the weight of the implant. The hollow structure may also increase the ease of attaching sutures. For example, straight suture holes need not be drilled, and the curved needle more readily passes through the integral attachment feature. The humeral replacement implant can be produced in any material (e.g., a metal) that is desired.

For example, a library can store the shapes of various humerus CT scans and processing can analyze the shapes of the humerus CT scans. After analyzing the humerus CT scans, the results can be divided into a plurality (e.g., five) of average sizes and shapes. However, more or less sizes or shapes can be used. The average sizes and shapes can be transferred to a CAD system. The CAD system can add patches with apertures to allow sutures to be passed through the implant to aid in attachment of tissue and ligaments to the implants.

The CAD system can be used to generate standard sized or personalized implants or implants on an as needed basis, reducing or eliminating inventory of round or rectangular metal bars kept as raw material inventory. For example, additive fabrication technology can be used to generate the implant.

Thus, one or more CT scans from a particular patient can be used to generate a patient-specific implant. Therefore, the implant would be personalized to a patient and potentially make the best use of the patient's bone stock. Specialized attachment features can be integrated into the patient specific implants to enhance the ability for the surgeon to attach tissue and ligaments during implantation.

Using the techniques described here, the humerus replacement implant can be better tailored to patient anatomy and can provide for bone preserving solutions. The humerus replacement implant can offer treatment options and flexibility to match unique patient needs. The average sizes and shapes for the implants, or the personalized implants, will results in more anatomic and physiologic reconstruction than current implant offerings. These characteristics enable a better match to patient needs and potentially better use of the patient's bone stock.

Using the techniques and designs described here, the humerus replacement implant may provide improved clinical outcomes. For example, the treatment solutions provided herein are a tangible improvement compared to the current standard of care. By providing patches for suturing, the implants offer improved interoperative speed and accuracy, and potentially, better security of the soft tissue attachment required in these types of reconstruction.

By eliminating the need for screws, screwdrivers, or separate porous coated parts to attach by providing the patches and/or areas of the implant that are porous, the implant will be more surgically convenient and result in significantly reduced operating room times, which benefits both the patient and the surgeon. The elimination of screws and separate porous coated parts allows for the elimination of protruding "bolt on" pieces that disturb the smooth contours over which musculature must act in order to function. The smooth contour of the present implant provides increased function of the musculature.

The humerus replacement implants of the present disclosure can include a hollow structure. The hollow structure provides a significantly lighter mass than comparable implant types that are hogged out from various pieces of solid bar stock. After receiving an implant, a patient's activities of daily living are limited to a few pounds. By reducing the overall load on the remaining musculature with a lighter implant, a better, more effective activities of daily living result are obtained because less muscle effort is used to move the arm and more of the available muscle effort can go to doing useful activities. Current implants are made from heavy, solid bar stock, therefore the hollow construction achieved by the implants of the present disclosure with a hollow design achieves an unmet patient need to reduce weight.

The implant, being made just additive metal technology, uses a powder and direct metal laser sintering (DMLS). The process and materials are relatively cheap, especially compared to solid bar stock of traditional implants, providing cost reductions to the patient.

The above discussion is intended to provide an overview of subject matter of the present patent application. It is not intended to provide an exclusive or exhaustive explanation of the invention. The description below is included to provide further information about the present patent application.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates a schematic of a plurality of humeral limbs of different sizes derived and/or reproduced from scans and/or one or more anatomical models in accordance with at least one example of the present disclosure.
FIG. 2A is a highly schematic illustration of an anterior proximal portion of a natural humerus with attachments for soft tissue displayed; FIG. 2A additionally shows a corresponding anterior proximal portion of a humeral limb replacement implant having patches corresponding in at least one of size, shape or location to at least some of the attachments shown in FIG. 2A to the viewers left.
FIG. 2B is a highly schematic illustration of an posterior proximal portion of a natural humerus with attachments for soft tissue displayed; FIG. 2A additionally shows a corresponding posterior proximal portion of a humeral limb replacement implant having patches corresponding in at least one of size, shape or location to at least some of the attachments shown in FIG. 2A to the viewers left.
FIG. 2C illustrates a schematic of a plurality of humeral limb replacement implant sizes that generally correspond to characteristics of the plurality of humeral limbs of FIG. 1 in accordance with at least one example of the present disclosure.
FIG. 3A illustrates a first view of a humeral limb replacement implant.
FIG. 3B illustrates a second view of the humeral limb replacement implant of FIG. 3A.
FIG. 3C illustrates a third view of the humeral limb replacement implant of FIG. 3A.
FIG. 3D illustrates a cross sectional view of the humeral limb replacement implant of FIG. 3A.
FIG. 4 illustrates a schematic view of segmented implants on a plurality of humeral limb replacement implant sizes.
FIG. 5A illustrates a first view of a first partial proximal humeral limb replacement implant.
FIG. 5B illustrates a second view of the first partial proximal humeral limb replacement implant of FIG. 5A.
FIG. 5C illustrates a third view of the first partial proximal humeral limb replacement implant of FIG. 5A.
FIG. 5D illustrates a second partial proximal humeral limb replacement implant.
FIG. 6A illustrates a first view of a third partial proximal humeral limb replacement implant.
FIG. 6B illustrates a second view of the third partial proximal humeral limb replacement implant of FIG. 6A.
FIG. 6C illustrates a third view of the third partial proximal humeral limb replacement implant of FIG. 6A.
FIG. 6D illustrates a fourth view of the third partial proximal humeral limb replacement implant of FIG. 6A.
FIG. 6E illustrates a fifth view of the third partial proximal humeral limb replacement implant of FIG. 6A.
FIG. 6F illustrates a sixth view of the third partial proximal humeral limb replacement implant of FIG. 6A.
FIG. 6G illustrates a seventh view of the third partial proximal humeral limb replacement implant of FIG. 6A.
FIG. 6H illustrates an eighth view of the third partial proximal humeral limb replacement implant of FIG. 6A.
FIG. 6I illustrates a ninth view of the third partial proximal humeral limb replacement implant of FIG. 6A.
FIG. 6J illustrates a tenth view of the third partial proximal humeral limb replacement implant of FIG. 6A.
FIG. 6K illustrates an eleventh view of the third partial proximal humeral limb replacement implant of FIG. 6A.
FIG. 6L illustrates a twelfth view of the third second partial proximal humeral limb replacement implant of FIG. 6A.
FIG. 6M illustrates a thirteenth view of the third partial proximal humeral limb replacement implant of FIG. 6A.
FIG. 7A illustrates a first view of a first partial distal humeral limb replacement implant.
FIG. 7B illustrates a second view of the first partial distal humeral limb replacement implant of FIG. 7A.
FIG. 7C illustrates a third view of the first partial distal humeral limb replacement implant of FIG. 7A.
FIG. 7D illustrates a second partial distal humeral limb replacement implant.
FIG. 7E illustrates a third partial distal humeral limb replacement implant.
FIG. 8A illustrates a first partial intercalary humeral limb replacement implant.
FIG. 8B illustrates a second partial intercalary humeral limb replacement implant.
FIG. 9A illustrates a first full humeral limb replacement implant assembly.
FIG. 9B illustrates a second full humeral limb replacement implant assembly.
FIG. 9C illustrates a third full humeral limb replacement implant assembly.
FIG. 10A illustrates a first view of a distal implant component for a humeral limb replacement implant assembly.
FIG. 10B illustrates a second view of the distal implant component of FIG. 10A for a humeral limb replacement implant assembly.
FIG. 11A illustrates a first view of a proximal implant component for a humeral limb replacement implant assembly.
FIG. 11B illustrates a second view of the proximal implant component of FIG. 11A for a humeral limb replacement implant assembly.
FIG. 12 illustrates an example of a method of manufacturing a limb replacement implant.
FIG. 13 shows a cross-section of a limb replacement implant having internal reinforcing structures that comprise ribs or ridges.
FIG. 14 shows a cross-section of a limb replacement implant having internal reinforcing structures that comprise honeycomb.

### DETAILED DESCRIPTION

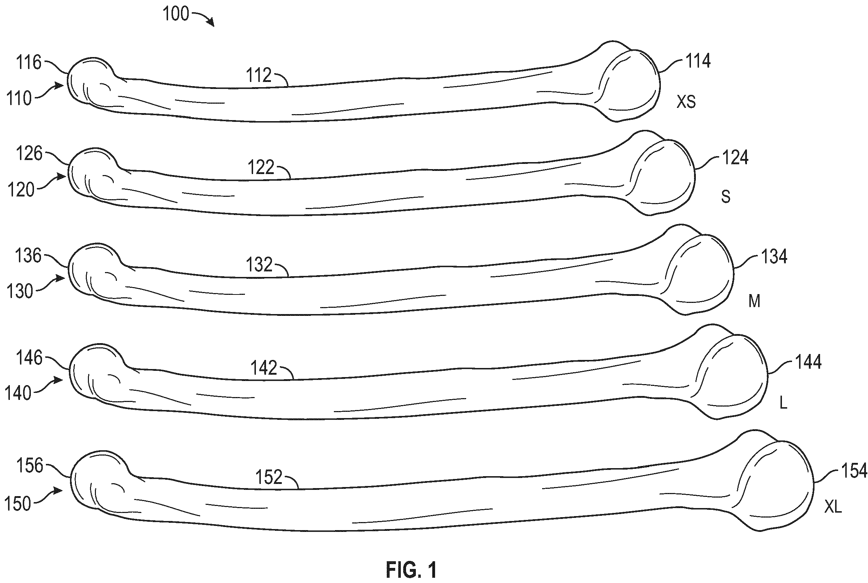

FIG. 1 is a schematic view of a plurality of humeral limbs 100 of different sizes derived and/or reproduced from scans and/or one or more anatomical models. As an example, the plurality of humeral limbs 100 can be software models generated from one or more humeral scans, which are analyzed and grouped together into the plurality of humeral limbs 100. Alternatively, or additionally, the plurality of humeral limbs 100 can be generated from anatomic modeling using a library such as the ZiBRA^{™} Anatomical Modeling System to analyze thousands of bones, both male and female, representing a diverse global population.

The plurality of humeral limbs 100 are shaped to simulate the natural anatomy of a typical patient based upon size. In general, each of the plurality of limbs 100 can include a proximal end, which is shaped to articulate with the scapula to form the shoulder j oint. The proximal end has a rounded, ball-like structure called the head. The head of the humerus is smooth and hemispherical, fitting into the shallow socket of the scapula (glenoid fossa) to form the glenohumeraljoint. The head connects to the body of the humerus via a constricted region called the anatomical neck. Each of the plurality of limbs 100 can additionally include a humeral shaft that extends between the proximal end and a distal end. The shaft has an elongate length and is generally cylindrical in shape having an oval or circular shape if viewed in cross-section taken perpendicular with a mechanical axis of the humerus. The shaft can include curvatures along a lateral side and a medial side, for example. Additionally, the shaft can have a slight anterior curvature and other curvature(s) along a lateral side and a medial side. The shaft contains the medullary cavity, which houses bone marrow. The distal end of the humerus forms the elbow joint with the bones of the forearm (radius and ulna). It consists of two prominent bony processes the trochlea and the capitulum.

FIG. 1 shows five different sizes for the plurality of humeral limbs 100. For example, the plurality of humeral limbs 100 may include an extra small (XS) size 110, a small (S) size 120, a medium (M) size 130, a large (L) size 140, and an extra large (XL) size 150. All the plurality of humeral limbs 100 may each include a shaft (e.g., XS shaft 112, S shaft 122, M shaft 132, L shaft 142, and XL shaft 152), a rounded proximal head (e.g., XS rounded proximal head 114, S rounded proximal head 124, M rounded proximal head 134, L rounded proximal head 144, and XL rounded proximal head 154), and a distal articular surface (e.g., XS distal articular surface 116, S distal articular surface 126, M distal articular surface 136, L distal articular surface 146, and XL distal articular surface 156).

The plurality of humeral limbs 100 may have different shaft lengths. These lengths can be determined based upon anatomic modeling data, can be arbitrary lengths or can be based upon other criteria (e.g., can be based upon standard sizing schemes, patient-specific factors, gender specific factors or other characteristics). For example, the computer program/model can generate an XS shaft 112 may have the shortest shaft length and the XL shaft 152 may have the longest shaft length. The computer program/model can generate S shaft 122 can be longer than the XS shaft 112, but shorter than the M shaft 132. The M shaft 132 can be longer than the S shaft 122, but shorter than the L shaft 142. The L shaft 142 can be longer than the M shaft 132, but shorter than the XL shaft 152. The cross-sectional shape of the shaft of the plurality of humeral limbs 100 may change along the length of the shaft as would the shape of a natural humerus. For example, portions of the shaft may have a substantially circular or ovoid cross-section and can change shape along a longitudinal length of the shaft. The cross-sectional shape of the shaft along the length of the shaft can be substantially similar to a humeral bone as the scans have an anatomic basis being generated from humeral bone scans, model(s), etc. According to another example, portions of the shaft may have other shapes as desired. For example, the shaft can have a substantially triangular cross section.

The plurality of humeral limbs 100 may have different shaft diameters or circumferences. For example, the XS shaft 112 may have the smallest shaft diameter or circumference, or a portion that has the smallest shaft diameter or circumference. The XL shaft 152 may have a portion of the shaft that has the largest shaft diameter or circumference. The S shaft 122 may have a portion of the shaft that has a larger diameter than the XS shaft 112 and may have a portion of the shaft that has a smaller diameter than the M shaft 132. The M shaft 132 may have a portion of the shaft that has a larger diameter or circumference than the S shaft 122 and may have a portion of the shaft that has a smaller diameter or circumference than the L shaft 142. The L shaft 142 may have a portion of the shaft that has a larger diameter or circumference than the M shaft 132 and may have a portion of the shaft that has a smaller diameter or circumference than the XL shaft 152. The diameter or circumference of the shaft may vary along the length of the shaft of the plurality of humeral limbs 100. Because the shaft diameter or circumference may vary along the length of the shaft, the plurality of humeral limbs 100 may have portions of the shaft with substantially similar diameters or circumferences between the various sizes.

The rounded proximal head (e.g., XS rounded proximal head 114, S rounded proximal head 124, M rounded proximal head 134, L rounded proximal head 144, and XL rounded proximal head 154) can be shaped substantially similar to the humeral head of a natural human bone. The XS rounded proximal head 114, S rounded proximal head 124, M rounded proximal head 134, L rounded proximal head 144, and/or XL rounded proximal head 154 can be substantially similar in size. The size of the rounded proximal head may increase from the XS rounded proximal head 114 to the S rounded proximal head 124, from the S rounded proximal head 124 to the M rounded proximal head 134, from the M rounded proximal head 134 to the L rounded proximal head 144, and/or from the L rounded proximal head 144 to the XL rounded proximal head 154.

The distal articular surface (e.g., XS distal articular surface 116, S distal articular surface 126, M distal articular surface 136, L distal articular surface 146, and XL distal articular surface 156) can be shaped substantially similar to a distal articular surface of a natural human bone. The XS distal articular surface 116, S distal articular surface 126, M distal articular surface 136, L distal articular surface 146, and/or XL distal articular surface 156 can be substantially similar in size. The size of the distal articular surface may increase from the XS distal articular surface 116 to the S distal articular surface 126, from the S distal articular surface 126 to the M distal articular surface 136, from the M distal articular surface 136 to the L distal articular surface 146, and/or from the L distal articular surface 146 to the XL distal articular surface 156.

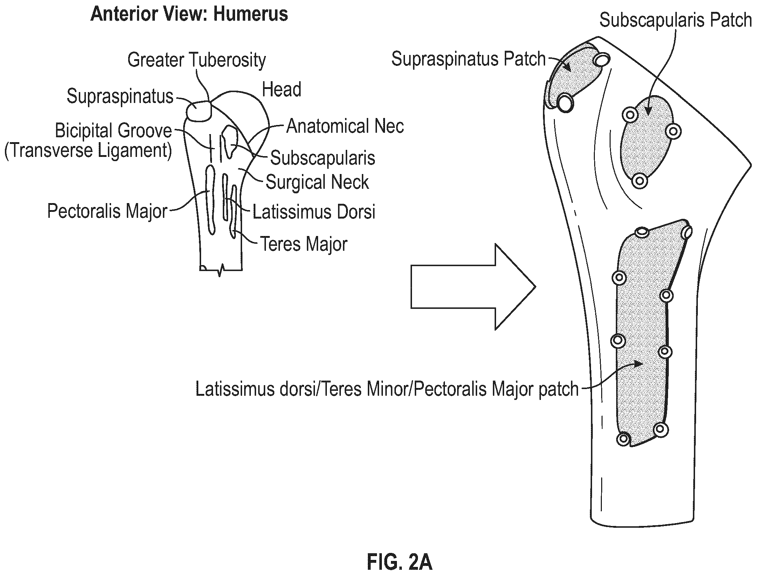

FIG. 2A shows an anterior view of a humerus with various anatomical attachments (also called insertions herein) shown. FIG. 2A additionally shows an exemplary limb replacement implant having a plurality of patches with at least some of the patches having at least one of a size, a shape and a position on corresponding to the anatomical attachments. For example, FIG. 2A shows the limb replacement implant with a supraspinatus patch corresponding in location, size and/or shape to the supraspinatus attachment shown to the viewers left. Additionally, the limb replacement implant includes a subscapularis patch corresponding in location, size and/or shape to the subscapularis attachment to the viewers left. FIG. 2A shows the limb replacement implant with a further patch corresponding in location, size and/or shape to a latissimus dorsi attachment, a teres minor attachment, and/or a pectoralis major attachment (shown in FIG. 2A to viewers left). Although only three patches are shown in FIG. 2A, further examples contemplate further distinct patches such as further patches on a distal portion of the limb replacement implant corresponding to anatomical attachment(s) of the natural humerus.

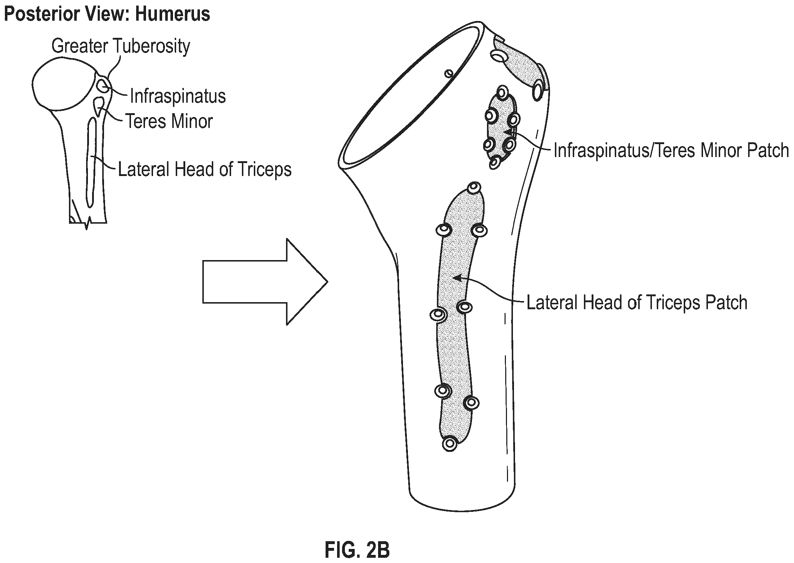

FIG. 2B illustrates a posterior of a natural humerus with various anatomical attachments (also called insertions herein) shown. FIG. 2A additionally shows the limb replacement implant of FIG. 2A further having a plurality of posterior patches with at least some of the posterior patches having at least one of a size, a shape and a position on corresponding to the anatomical attachments of FIG. 2B. For example, FIG. 2B shows the limb replacement implant with an infraspinatus and/or teres minor patch corresponding in location, size and/or shape to the infraspinatus and/or teres minor attachment(s) shown to the viewers left. Additionally, the limb replacement implant includes a lateral head of triceps patch corresponding in location, size and/or shape to the lateral head of triceps attachment to the viewers left. Although only two patches are shown in FIG. 2B, further examples contemplate further distinct patches such as further patches on a distal portion of the limb replacement implant corresponding to anatomical attachment(s) of the natural humerus.

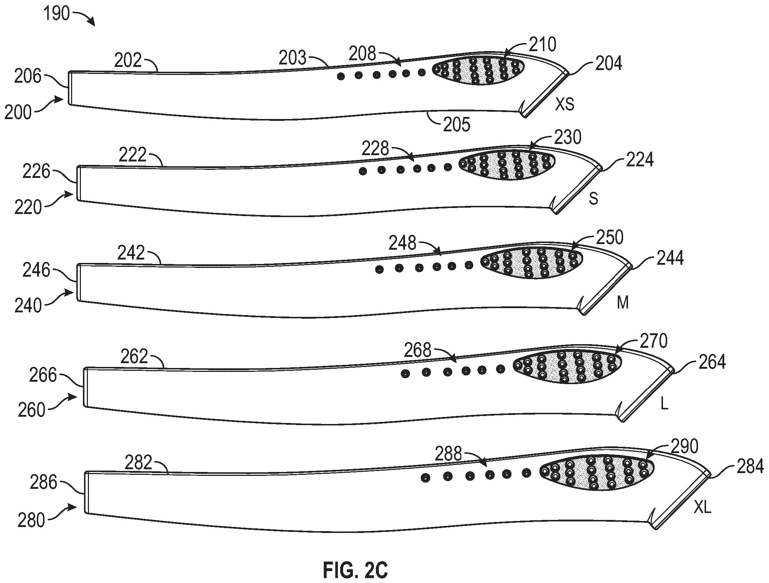

FIG. 2C illustrates a schematic of a plurality of humeral limb replacement implants 190 having different sizes corresponding to the sizes of the plurality of humeral limbs 100 (see FIG. 1). The plurality of humeral limbs 100 can thus be converted to the plurality of humeral limb replacement implants 190. For example, five sizes can be determined. However, different numbers of implants 190 can be applicable. The number of humeral limb replacement implants 190 may depend on the number of humeral limbs 100 as determined by modeling, analysis, anatomic characteristics, etc. Additionally, or alternatively, the plurality of humeral limbs 100 can be converted into a different number of humeral limb replacement implants 190. For example, more humeral limb replacement implants 190 can be created by interpolating between the plurality of humeral limbs 100. As another example, less humeral limb replacement implants 190 can be created by converting the plurality of humeral limbs 100 with the highest likelihood of use. The plurality of humeral limb replacement implants 190 can be created prior to fabrication using computer-aided design (CAD). When converting the plurality of humeral limbs 100 to the plurality of humeral limb replacement implants 190, the edges of the implant can be smoothed while maintaining the look and shape of the humerus bone. This shape is configured to substantially correspond to a shape of a natural humerus such as by having the shaft of the implants 190 be elongate and have at least one curvature proximal-distal, elongate with the proximal portion widened and otherwise shaped to simulate at least a portion of a head of the humerus, elongate and having two or more curvatures along a lateral side surface 203 thereof (the lateral surface 203 is only indicated in the context of the implant 200) and/or elongate and having two or more curvatures along a medial side surface 205 thereof (the medial side surface 205 is only indicated in the context of the implant 200). Additionally, the shaft of the implants 190 can have slight anterior curvature similar to that of a natural humerus.

For example, the plurality of humeral limb replacement implants 190 may include an extra small (XS) humeral limb replacement implant 200, a small (S) humeral limb replacement implant 220, a medium (M) humeral limb replacement implant 240, a large (L) humeral limb replacement implant 260, and an extra large (XL) humeral limb replacement implant 280. All of the plurality of humeral limb replacement implants 190 can each include a shaft (e.g., XS shaft 202, S shaft 222, M shaft 242, L shaft 262, and XL shaft 282), a proximal end (e.g., XS proximal end 204, S proximal end 224, M proximal end 244, L proximal end 264, and XL proximal end 284), and a distal end (e.g., XS distal end 206, S distal end 226, M distal end 246, L distal end 266, and XL distal end 286). The plurality of humeral limb replacement implants 190 may also include a shaft patch (e.g., XS shaft patch 208, S shaft patch 228, M shaft patch 248, L shaft patch 268, and XL shaft patch 288) and/or a proximal patch (e.g., XS proximal patch 210, S proximal patch 230, M proximal patch 250, L proximal patch 270, and XL proximal patch 290). The examples of FIG. 2C only illustrate some of the possible patches and possible locations for patches. The number, shape, size and location of the patches can correspond to a number of insertions (attachments) with soft tissue on a natural humerus, for example. Thus, the humeral limb replacement implants 190 can have between five and twenty-five patches according to some examples. However, larger patches and/or less patches than would naturally correspond to insertions on the natural humerus are also contemplated. Thus, the humeral limb replacement implants 190 can have less than five patches according to some examples.

The plurality of humeral limb replacement implants 190 may have different shaft lengths. The length of the shafts of the plurality of humeral limb replacement implants 190 can be substantially similar to the length of the shafts of the plurality of humeral limbs 100. For example, the XS shaft 202 length can be substantially similar to the XS shaft 112 length, the S shaft 222 length can be substantially similar to the S shaft 122 length, the M shaft 242 length can be substantially similar to the M shaft 132 length, the L shaft 262 length can be substantially similar to the L shaft 142 length, and the XL shaft 282 length can be substantially similar to the XL shaft 152 length. The shafts of the plurality of humeral limb replacement implants 190 may increase in length from the XS shaft 202 to the S shaft 222, from the S shaft 222 to the M shaft 242, from the M shaft 242 to the L shaft 262, and from the L shaft 262 to the XL shaft 282.

The cross-sectional shape of the shaft of the plurality of humeral limb replacement implants 190 may change along the elongate length of the shaft. For example, portions of the shaft may have a substantially circular cross section, a substantially triangular cross section, a substantially oval-shaped cross section. The cross-sectional shape of the shaft along the length of the shaft can be substantially similar to the plurality of humeral limbs 100, which are substantially similar to a humeral bone. Additionally or alternatively, the shafts, or portions thereof, of the plurality of humeral limb replacement implants 190 can be smoothed, rounded, and/or curved including along the medial side, the lateral side and proximal-distal as previously discussed. The smooth and/or rounded contour may allow musculature to interface more easily with the humeral limb replacement and increase function. The smooth and/or rounded contour may create a more pain free result for a patient.

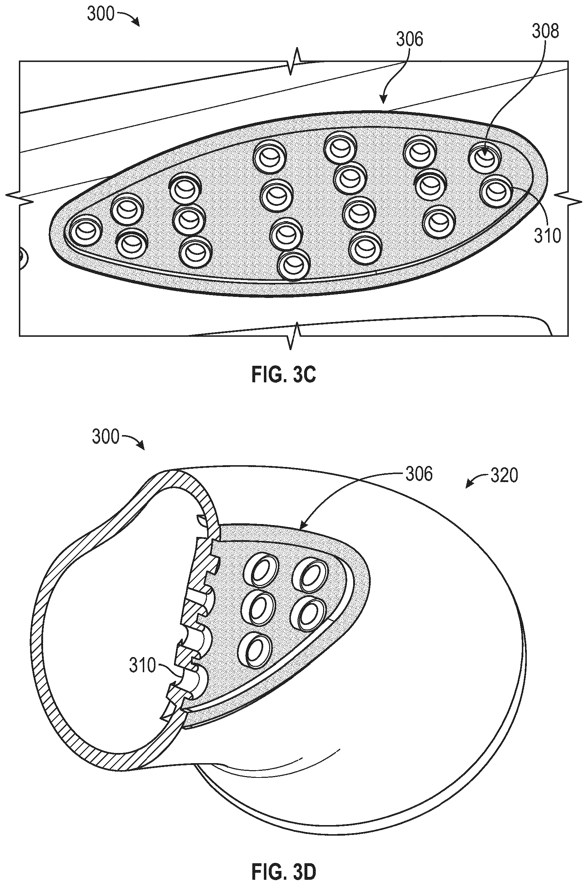

The plurality of humeral limb replacement implants 190 may have different shaft diameters or circumferences. For example, the XS shaft 202 may have the smallest shaft diameter or circumference, or a portion that has the smallest shaft diameter or circumference. The XL shaft 282 may have a portion of the shaft that has the largest shaft diameter or circumference. The S shaft 222 may have a portion of the shaft that has a larger diameter than the XS shaft 202 and may have a portion of the shaft that has a smaller diameter than the M shaft 242. The M shaft 242 may have a portion of the shaft that has a larger diameter or circumference than the S shaft 222 and may have a portion of the shaft that has a smaller diameter or circumference than the L shaft 262. The L shaft 262 may have a portion of the shaft that has a larger diameter or circumference than the M shaft 242 and may have a portion of the shaft that has a smaller diameter or circumference than the XL shaft 282. The diameter or circumference of the shaft may vary along the length of the shaft of the plurality of humeral limb replacement implants 190. Because the shaft diameter or circumference may vary along the length of the shaft, the plurality of humeral limb replacement implants 190 may have portions of the shaft with substantially similar diameters or circumferences between the various sizes. Additionally, or alternatively, the shaft 202, 222, 242, 262, 282 can have at least one of: hollow shape (see FIG. 3D as example), elongate and having at least one curvature proximal-distal (such at least one curvature simulates the curvature proximal-distal of the natural shaft of the humerus as shown in FIG. 1), elongate with the proximal portion configured to simulate at least a portion of a head of the humerus (again see FIG. 1), elongate and having two or more curvatures along the lateral side surface 203 thereof, elongate and having two or more curvatures along the medial side surface 205 thereof.

Even with a limited number of sizes 190, the implants 190 can be scaled up or down based on specific anatomy of a patient. When converting the plurality of humeral limbs 100 to the plurality of humeral limb replacement implants 190, elbow and proximal implant interfaces can be added. For example, the proximal end can be configured to be a proximal implant interface, such that a proximal implant can be coupled to the proximal end. For example, the distal end can be configured to be an elbow implant interface, such that an elbow implant, or a component thereof, can be coupled to the distal end.

The proximal end (e.g., XS proximal end 204, S proximal end 224, M proximal end 244, L proximal end 264, and XL proximal end 284) of the shaft can be substantially circular. The proximal end can be substantially flat. The proximal end can be substantially flat to couple a proximal implant to the proximal end while maintaining a substantially smooth exterior at the interface. The flat surface of the proximal portion can be positioned such that a central axis of the shaft and the central axis of the flat portion create an angle. The XS proximal end 204, S proximal end 224, M proximal end 244, L proximal end 264, and/or XL proximal end 284 can be substantially similar in size. The size of the proximal end may increase from the XS proximal end 204 to the S proximal end, from the S proximal end 224 to the M proximal end 244, from the M proximal end 244 to the L proximal end 264, and/or from the L proximal end 264 to the XL proximal end 284.

The distal end (e.g., XS distal end 206, S distal end 226, M distal end 246, L distal end 266, and XL distal end 286) can be substantially oval-shaped. The distal end can be substantially flat. The distal end can be substantially flat to couple a distal implant to the distal end while maintaining a substantially smooth exterior at the interface. The flat surface of the distal end can be positioned such that a central axis of the shaft and the central axis of the flat portion are parallel. The XS distal end 206, S distal end 226, M distal end 246, L distal end 266, and/or XL distal end 286 can be substantially similar in size. The size of the distal end may increase from the XS distal end 206 to the S distal end 226, from the S distal end 226 to the M distal end 246, from the M distal end 246 to the L distal end 266, and/or from the L distal end 266 to the XL distal end 286.

The shaft patch (e.g., XS shaft patch 208, S shaft patch 228, M shaft patch 248, L shaft patch 268, and XL shaft patch 288) and/or the proximal patch (e.g., XS proximal patch 210, S proximal patch 230, M proximal patch 250, L proximal patch 270, and XL proximal patch 290) can be a portion of porous material. The patches (e.g., shaft patch and/or proximal patch) can be an array of apertures to receive soft sutures. The patches (e.g., shaft patch and/or proximal patch) may include a portion of porous material surrounded by a plurality of apertures. Additionally, or alternatively, the patches (e.g., shaft patch and/or proximal patch) may include a plurality of apertures within a portion of porous material. The number of apertures and location of apertures can vary from embodiment to embodiment and can vary based upon anatomic or other criteria. Thus, the number of apertures can be between ten and seventy according to some examples. However, implants with more than seventy apertures or less than ten apertures are also contemplated. The patches (e.g., shaft patches and/or proximal patches) may align with zones specific to anatomic muscle attachment areas, for example. The patches and/or apertures (e.g., shaft patches and/or proximal patches) can be substantially oval-shaped, linear, rectangular, circular, or another shape as needed to align with muscle attachment areas and/or to facilitate anchoring to the implant with suture.

The XS shaft patch 208, S shaft patch 228, M shaft patch 248, L shaft patch 268, and/or XL shaft patch 288 can be substantially similar in size. The shaft patches of the plurality of humeral limb replacement implants 190 can be scaled up or down depending on the implant size. This sizing up or down of the shaft patches may include changing a size of a portion of porous material, changing a number of apertures, and/or changing the size of the apertures. If the shaft patch includes a portion of porous material, the size of the portion of porous material may increase from the XS shaft patch 208 to the S shaft patch 228, from the S shaft patch 228 to the M shaft patch 248, from the M shaft patch 248 to the L shaft patch 268, and/or from the L shaft patch 268 to the XL shaft patch 288. If the shaft patch includes a plurality of apertures, the number of apertures may increase from the XS shaft patch 208 to the S shaft patch 228, from the S shaft patch 228 to the M shaft patch 248, from the M shaft patch 248 to the L shaft patch 268, and/or from the L shaft patch 268 to the XL shaft patch 288.

The XS proximal patch 210, S proximal patch 230, M proximal patch 250, L proximal patch 270, and/or XL proximal patch 290 can be substantially similar in size. The proximal patches of the plurality of humeral limb replacement implants 190 can be scaled up or down depending on the implant size. This sizing up or down of the proximal patches may include changing a size of a portion of porous material, changing a number of apertures, and/or changing the size of the apertures. If the proximal patch includes a portion of porous material, the size of the portion of porous material may increase from the XS proximal patch 210 to the S proximal patch 230, from the S proximal patch 230 to the M proximal patch 250, form the M proximal patch 250 to the L proximal patch 270, and/or from the L proximal patch 270 to the XL proximal patch 290. If the proximal patch includes a plurality of apertures, the number of apertures may increase from the XS proximal patch 210 to the S proximal patch 230, from the S proximal patch 230 to the M proximal patch 250, form the M proximal patch 250 to the L proximal patch 270, and/or from the L proximal patch 270 to the XL proximal patch 290.

The plurality of humeral limb replacement implants 190 can become the foundation for additional implants or features. The implants 190 can be made using any material (e.g., metal) and be made using additive metal technologies. As such, the plurality of humeral limb replacement implants 190 become the `raw material' for all of the implant parts of a humeral limb replacement implant system, replacing the traditional round bar stock.

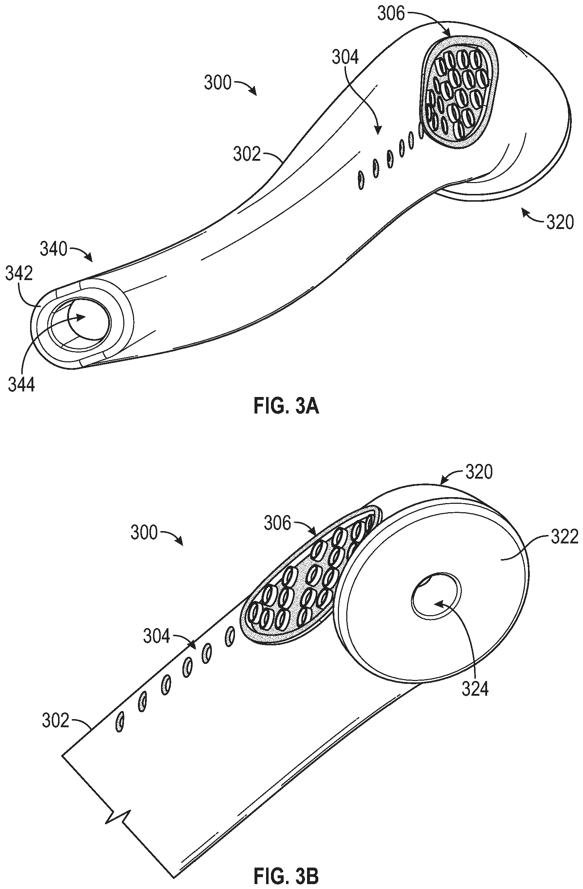

FIGS. 3A-3D illustrate various views of a humeral limb replacement implant 300. FIG. 3A shows an anterior side and a distal end 340 with the distal end having a connection feature for coupling with a second prosthesis that simulates an elbow. This connection feature allows the implant 300 to be connected with additional prosthesis components as an assembly to reproduce an arm of the patient. FIG. 3B shows a proximal portion of the humeral limb replacement implant 300 having a connection feature for coupling with a third prosthesis that simulates the head of a natural humerus. FIG. 3C shows a proximal patch 306 with apertures 310. FIG. 3D is a partial cross-section through a portion of the humeral limb replacement implant 300 at the proximal patch 306 showing the implant 300 can be hollow within an interior according to some examples.

As shown in FIGS. 3A and 3B, the humeral limb replacement implant 300 includes a shaft 302, a proximal end 320, and a distal end 340. The humeral limb replacement implant 300 may also include a shaft patch 304 and/or the proximal patch 306.

The shaft 302 can be formed to follow a natural shape of a humeral bone. The length of the shaft 302 of humeral limb replacement implant 300 can be determined based on the size selected for the humeral limb replacement implant 300. The cross-sectional shape of the shaft 302 of the humeral limb replacement implant 300 may change along the length of the shaft 300. For example, portions of the shaft may have a substantially circular cross section, a substantially triangular cross section, and/or a substantially oval-shaped cross section. In an example, the exterior of the shaft 302, or portions thereof, can be smoothed and/or rounded. The smooth and/or rounded contour may allow musculature to interface more easily with the humeral limb replacement implant 300 and provide an increase in function of the humeral limb replacement implant 300. The smooth and/or rounded contour of the shaft 302 may create a more pain free result for a patient because the implant simulates nature bone better than alterative humeral replacements. In an example, a diameter or circumference of the shaft 302 may vary along the length of the shaft 302 of the humeral limb replacement implant 300. The humeral limb replacement implant 300 can be made using an additive metal manufacturing. For example, additive metal manufacturing may use fine, metal powders to create the humeral limb replacement implant 300. The material can be deposited layer by layer to create an object.

The shaft 302 can be hollow. For example, the shaft 302 may have a wall with a thickness of approximately 1.5 mm. A hollow shaft 302 may create a lighter humeral limb replacement implant 300. If the weight of the humeral limb replacement implant 300 is reduced, the patient can be able to bear additional weight in the arm with the humeral limb replacement implant 300. Specifically, after a humeral limb replacement implant surgery, a patient can be limited to a certain maximum amount of weight bearing. While the maximum amount of weight bearing will not change, by reducing the weight required to lift the humeral limb replacement implant, additional weight can be allocated to lifting objects. In an example, the shaft 302 can be made separately in parts and then joined together. For example, the shaft 302 can be fabricated in a proximal half and a distal half and welded together in the middle. The shaft 302 can be made in halves to allow unfused powder to be removed.

The proximal end 320 of the shaft 302 may have a substantially circular cross section. The proximal end 320 may have a greater width, diameter, or circumference than the shaft 302. The proximal end 320 may include a proximal surface 322. The proximal surface 322 can be substantially flat. The proximal surface 322 can be positioned such that a central axis of the shaft 302 and a central axis of the proximal surface 322 create an angle. The proximal surface 322 can be substantially flat to couple a proximal implant to the proximal end 320 while maintaining a substantially smooth exterior at the interface. The proximal surface 322 may include a proximal aperture 324 along the central axis of the proximal surface 322. The proximal aperture 324 may receive the proximal implant to secure the proximal implant to the proximal end 320 of the humeral limb replacement implant 300. For example, the proximal aperture 324 can include a snap configuration. The proximal implant received by the proximal aperture 324 can be a humeral tray or a humeral head.

The distal end 340 may have a substantially oval-shaped cross section. The distal end 340 may have a greater width, diameter, or circumference than the shaft 302. The distal end 340 may include a distal surface 342. The distal surface can be substantially flat. The distal surface 342 can be substantially flat to couple a distal implant to the distal end 340 while maintaining a substantially smooth exterior at the interface. The distal surface 342 of the distal end 340 can be positioned such that a central axis of the shaft 302 and the central axis of the distal surface are parallel substantially along a portion of the shaft 302. The distal surface 342 may include a distal aperture 344 along the central axis of the distal surface 342. The distal aperture 344 may receive the distal implant to secure the distal implant to the distal end 340 of the humeral limb replacement implant 300. For example, the distal aperture 344 can include a snap configuration. The distal implant received by the distal aperture 344 can be an elbow implant.

The shaft patch 304 can be positioned along a portion of the shaft 302. The shaft patch 304 may facilitate attachment of muscle, ligaments, and/or other tissue. For example, the shaft patch 304 can be a porous structure that facilitates tissue ingrowth and remodeling and vascularization. The porous structure can be created by a porous design and/or a porous material.

The shaft patch 304 can be shaped, sized, and positioned to facilitate good reconstruction by aligning with zones specific to anatomic attachment areas. The location/size/shape of the individual patches (e.g., shaft patch 304) and/or of porous/suture holes can be derived from their anatomical counterparts on the actual human physiology. The shaft patch 304 can be substantially oval-shaped, linear, rectangular, circular, or another shape as needed to align with the anatomic attachment areas. In an example, the characteristics (e.g., shape, size, and/or position) of the shaft patch 304 can be customized to meet the specific needs of a patient. The shaft patch 304 may include shaft patch attachment holes 308. The shaft patch attachment holes 308 may receive soft sutures. The shaft patch 304 may include a portion of porous structure surrounded by shaft patch attachment holes. Additionally or alternatively, the shaft patch 304 may include shaft patch attachment holes 308 within a portion of porous structure. The shaft patch 304 can be recessed in the shaft 302. For example, the shaft patch 304 can be recessed to allow shaft patch attachment holes 308 to receive sutures while still maintaining a substantially flush exterior of the shaft 302.

The proximal patch 306 can be positioned at a proximal end 320 of the shaft 302. The proximal patch 306 may facilitate attachment of muscle, ligaments, and/or other tissue. For example, the proximal patch 306 can be a porous structure that facilitates tissue ingrowth and remodeling and vascularization. The porous structure can be created by a porous design and/or a porous material. The proximal patch 306 can be shaped, sized, and positioned to facilitate good reconstruction by aligning with zones specific to anatomic attachment areas. The proximal patch 306 can be substantially oval-shaped, linear, rectangular, circular, or another shape as needed to align with the anatomic attachment areas. In an example, the characteristics (e.g., shape, size, and/or position) of the proximal patch 306 can be customized to meet the specific needs of a patient.

The proximal patch 306 may include a proximal patch attachment holes 308. The proximal patch attachment holes 308 may receive soft sutures. The proximal patch 306 may include a portion of porous structure surrounded by proximal patch attachment holes 308. Additionally, or alternatively, the proximal patch 306 may include proximal patch attachment holes 308 within a portion of porous structure. In an example, proximal patch attachment holes 308 may have proximal patch attachment hole edges 310. The proximal patch attachment hole edges 310 can be a raised boss edge. The proximal patch attachment hole edges 310 may aid in securing the sutures. The proximal patch attachment hole edges 310 can be located on an exterior side of the proximal patch attachment holes 308. Additionally, or alternatively, the proximal patch attachment hole edges 310 can be located on an interior side of the proximal patch attachment holes 308. If the shaft 302 is hollow, the proximal patch attachment hole edges 310 may aid in securing the sutures (e.g., a JuggerKnot^{®} suture or other suture or suture anchor such as one or more suture products offered by Zimmer Biomet, Inc. of Warsaw Indiana) by securing the knot of a suture. The proximal patch 306 can be recessed in the shaft 302. For example, the proximal patch 306 can be recessed to allow shaft patch attachment holes 308 to receive sutures while still maintaining a substantially flush exterior of the shaft 302. As another example, the proximal patch 306 can be recessed to allow proximal patch attachment hole edges 310 to extend from the proximal patch attachment holes 308 while maintaining a substantially flush exterior of the shaft 302.

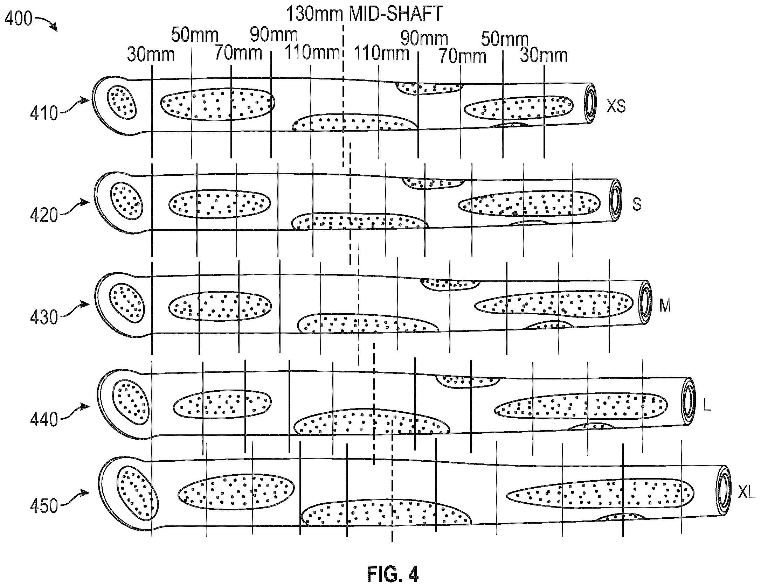

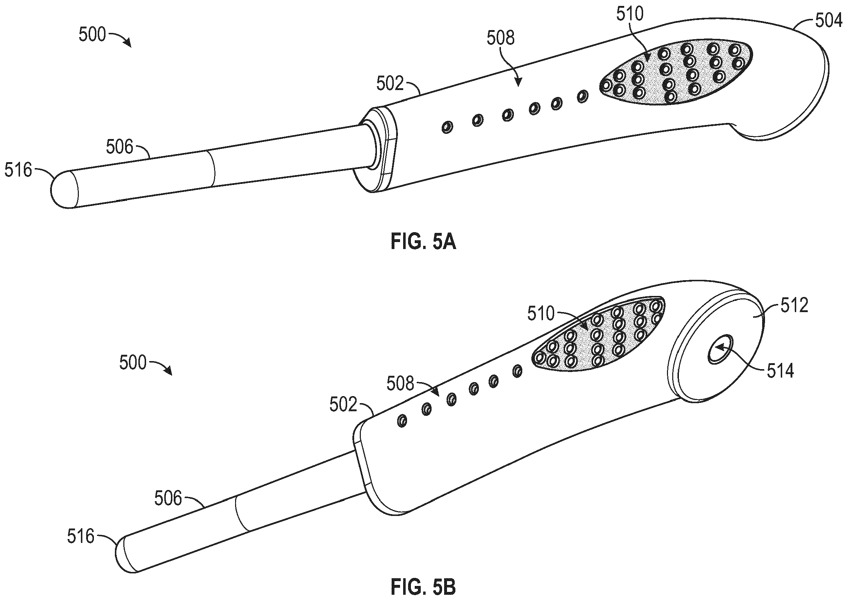

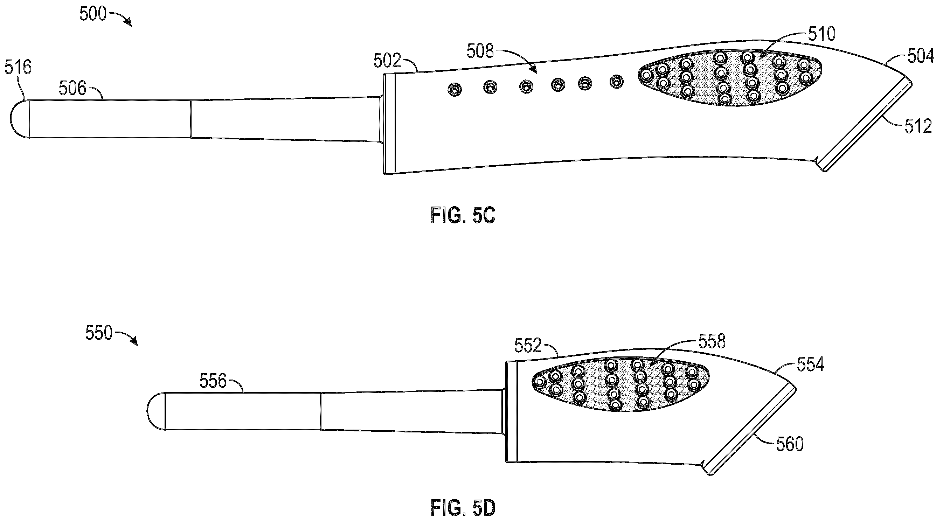

FIG. 4 illustrates a schematic view of a plurality of implants 400 of different sizes with patches at different distances indicated from the mid-line or other anatomical location. Lines indicating the different distances (e.g., 30 mm, 50 mm, etc.) can be utilized to identify segments of a plurality of humeral limb replacement implant sizes. These segments can be utilized in modifying the plurality of implants 400 design to that of a partial humeral implant as further illustrated in FIGS. 5A-8B to persevere existing acceptable bone.

The plurality of humeral limb replacement implants 190 (from FIG. 2C) can be segmented using computer processing into a plurality of segment lengths. For example, the segmented implants 400 may include an extra small (XS) segmented humeral limb replacement implant 410, a small (S) segmented humeral limb replacement implant 420, a medium (M) segmented humeral limb replacement implant 430, a large (L) segmented humeral limb replacement implant 440, and an extra large (XL) segmented humeral limb replacement implant 450.

For example, the plurality of segment lengths may include a midshaft segment. Each half of the midshaft segment can be further segmented into six segments of substantially equal size. For example, segments can be 20 mm to 30 mm in length. In an example, the distal most segment and/or the proximal most segment may have a longer length. For example, the distal most segment and/or the proximal most segment may have a length of 30 mm, while other segments have a length of 20 mm. As another example, as the size of the segmented implants 400 increases, the length of the segments may increase. Alternatively, the length of the segments may remain the same as the size of the segmented implants 400 increases, such that larger size implants have more segments. In another example, the segment length of the humeral limb replacement implant can be customized such that any length of humeral limb replacement implant segment can be created.

Using the segmented implants 400, a humeral limb replacement implant can be fabricated so as to be segmented to create a partial humeral limb replacement implant that can be coupled to an existing portion of a natural humerus of the patient. Rather than an entire humeral limb replacement implant that replaces the entirety (or substantially all) of the natural humerus, only a portion of the natural humerus is removed and is replaced with a stem to be fitting within the remaining natural humerus of the patient. The humeral limb replacement implant can be segmented from either the proximal side and/or the distal side, as patients often do not need a full humerus replacement. For example, any length of a humeral limb replacement implant can be created based on the length of humeral limb replacement implant needed by the patient.

FIGS. 5A-5C illustrate various views of a first partial proximal humeral limb replacement implant 500. The partial proximal humeral limb replacement implant 500 can be used to replace just a proximal portion of a patient's humerus. A partial humeral replacement, such as the first partial proximal humeral limb replacement implant 500 can use the proximal structure shape that is truncated with addition of a stem to match patient's needs in preserving natural bone. A distal portion of the implant 500 can be shaped as the stem or other feature to couple with existing natural distal portion of the natural humerus. The partial proximal humeral limb replacement implant 500 includes a shaft 502, a proximal end 504, and a stem 506. The humeral limb replacement implant 500 may also include a shaft patch 508 and/or a proximal patch 510.

The shaft 502 can be substantially similar to shaft 302 (see FIG. 3). However, shaft 502 can be truncated to include only a proximal portion. By truncating with the proximal portion, the shaft 502 includes a proximal end 504. The length of the shaft 502 can be predetermined. As another example, the length of the shaft 502 can be customized to be patient specific. The proximal end 504 of the shaft 502 can be substantially similar to proximal end 320 (see FIG. 3). The proximal end 504 may include a proximal surface 512 and a proximal aperture 514. The proximal surface 512 can be substantially similar to proximal surface 322 (see FIG. 3). The proximal aperture 514 can be substantially similar to proximal aperture 324 (see FIG. 3). The shaft patch 508 can be substantially similar to shaft patch 304 (see FIG. 3). The proximal patch 510 can be substantially similar to proximal patch 306 (see FIG. 3).

The stem 506 may extend distally from the shaft 502. During a humeral limb replacement procedure, the stem 506 is inserted into humerus while the shaft 502 abuts the humerus. The length of the stem 506 can be determined once the length of the shaft 502 is determined. For example, the overall length of the humeral limb replacement implant 500 can be determined first (e.g., based on an overall size of the patient's humerus), then the length of the shaft 502 can be determined (e.g., based on the proximal portion of the patient's humerus that needs to be replaced), and then the length of the stem 506 is determined (e.g., the remaining length based on the overall length and the length of the shaft 502). The stem 506 may have a substantially circular cross section. As another example, the stem 506 may have an oval-shaped cross section. The shape of the cross section of the stem 506 may vary along the length of the stem 506. The size of the cross section of the stem 506 may vary along the length of the stem 506. For example, the stem 506 may taper (e.g., decrease in diameter, circumference, and/or width) from a proximal end to a distal end 516. The distal end 516 can be rounded. As another example, the distal end 516 can be pointed to aid in driving the humeral limb replacement implant 500 into the humerus.

FIG. 5D illustrates a second partial proximal humeral limb replacement implant 550. The partial humeral replacement, such as the second partial proximal humeral limb replacement implant 550 can use the proximal structure shape of the total implants of FIGS. 2-4 but can be truncated and has a stem to meet the patient's needs. The implant 550 can be relatively more bone preserving having a shorter proximal extent than the implant 500 of FIGS. 5A-5C. The partial proximal humeral limb replacement implant 550 includes a shaft 552, a proximal end 554, and a stem 556.

The shaft 552 can be substantially similar to shaft 302 (see FIG. 3). The length of the shaft 552 can be predetermined. As another example, the length of the shaft 552 can be customized to be patient specific. For example, shaft 552 can be truncated to include only a proximal portion. As another example, the proximal portion of the shaft 552 may include only a proximal end 504. For example, the shaft 552 can be shorter than the shaft 502 (see FIGS. 5A-5C).

The stem 556 can be substantially similar to the stem 506 (see FIGS. 5A-5C). However, as the shaft 556 is shorter than the shaft 502, the stem 556 can be longer than the stem 506, such that the second partial proximal humeral limb replacement implant 550 and the first partial proximal humeral limb replacement implant 500 are substantially similar in length. In another example, the stem 556 can be a different length as the stem 506. For example, the first partial proximal humeral limb replacement implant 500 and the second partial proximal humeral limb replacement implant 550 can be different sizes of humeral limb replacement implants. As another example, a width of the stem 556 can be substantially similar to stem 506, or can be different than stem 506, depending on the selected size of the corresponding humeral limb replacement implant.

The proximal end 554 of the shaft 552 can be substantially similar to proximal end 320 (see FIG. 3). The proximal end 554 may include a proximal surface 560 and a proximal aperture (not shown). The proximal surface 560 can be substantially similar to proximal surface 322 (see FIG. 3). The proximal aperture can be substantially similar to proximal aperture 324 (see FIG. 3). The humeral limb replacement implant 550 may also include a proximal patch 558. The proximal patch 558 can be substantially similar to proximal patch 306 (see FIG. 3). Due to the truncation of the shaft 552, the shaft 552 does not include a shaft patch.

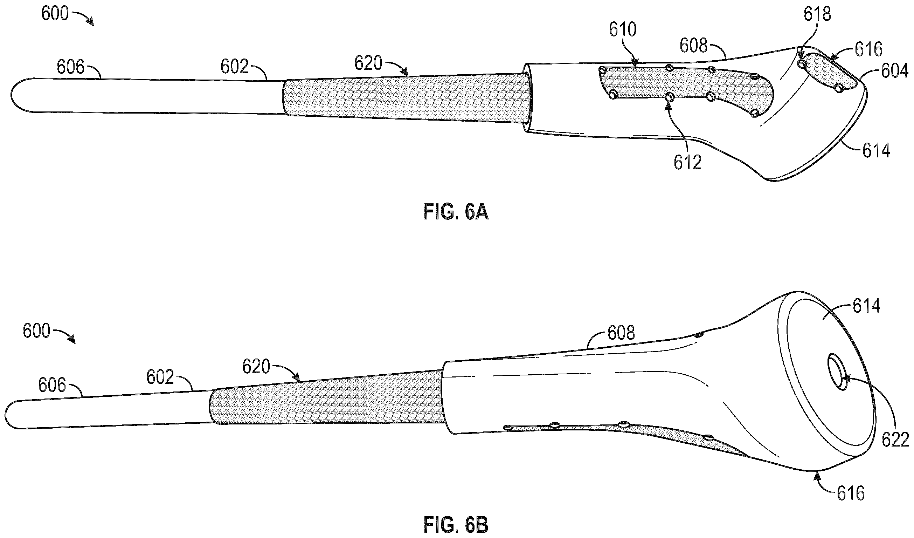

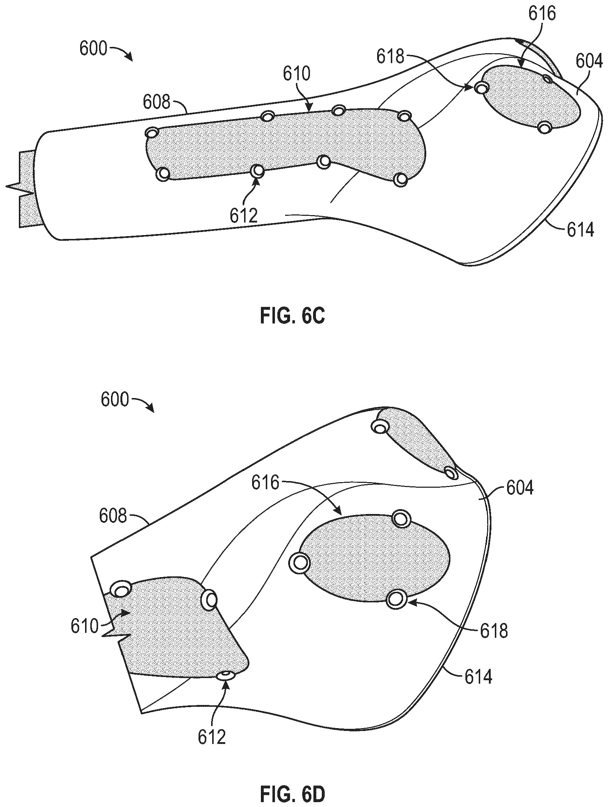

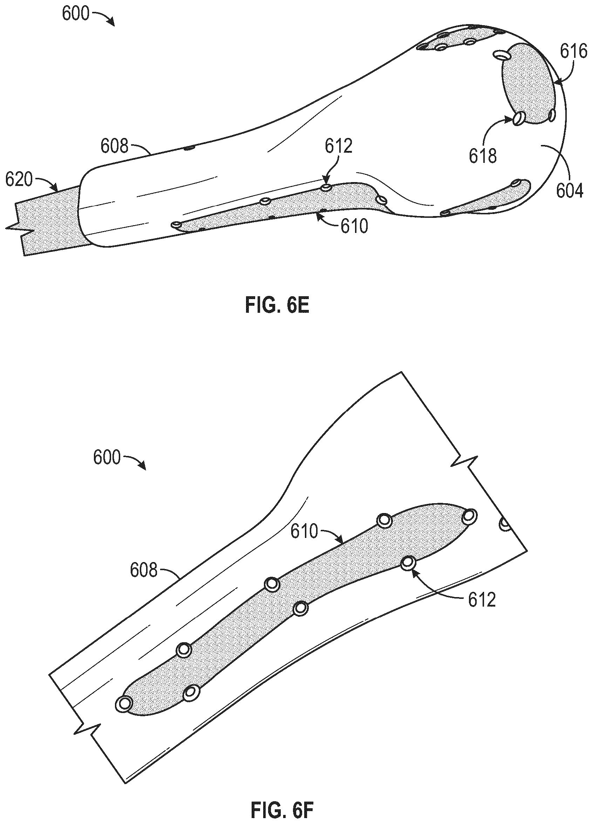

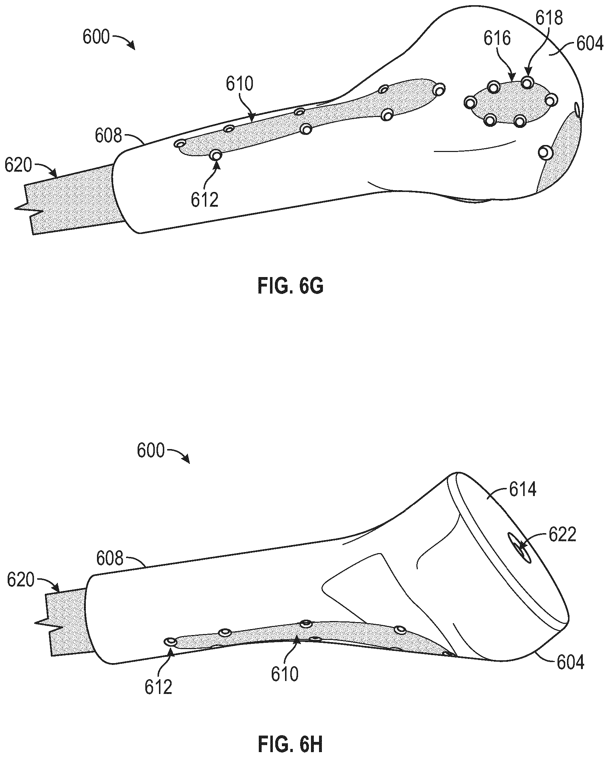

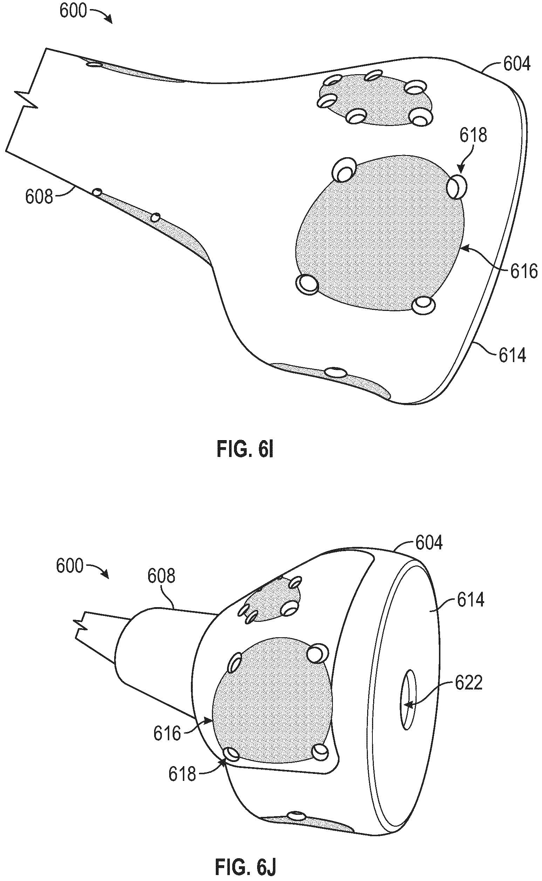

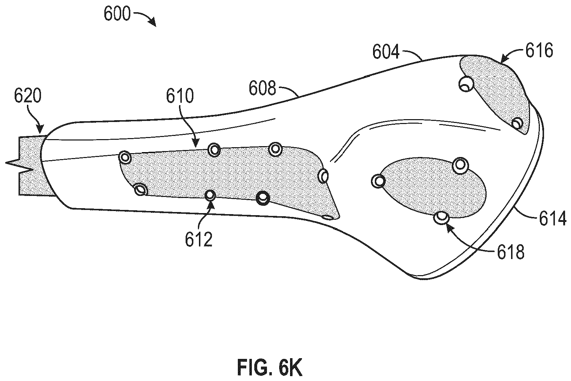

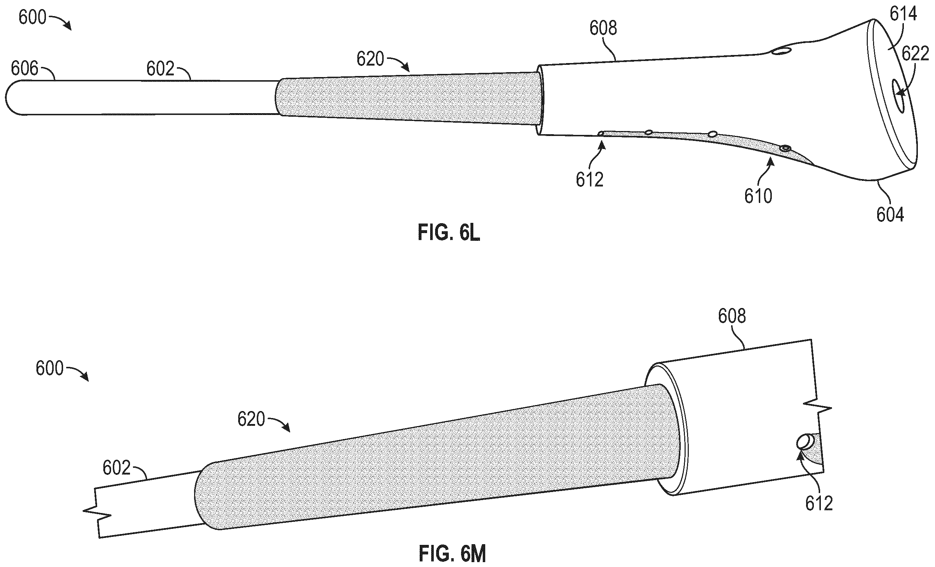

FIGS. 6A-6M illustrate various aspects of a third partial proximal humeral limb replacement implant 600 including patches along an intermediate stem thereof, patches along several parts of a proximal portion thereof, apertures adjacent one or more of the patches, etc. The humeral limb replacement implant 600 includes a stem 602 and a shaft 608. The stem 602 includes a distal end 606 and the shaft 608 includes a proximal end 604. The humeral limb replacement implant 600 can be made using an additive manufacturing process. For example, additive metal manufacturing may use fine, metal powders fused by heat from a laser (or other deposition type processes) to create the humeral limb replacement implant 600. The material can be deposited layer by layer to create an object. The humeral limb replacement implant 600 can be constructed as a single piece. In another example, the humeral limb replacement implant 600 can be made in multiple sections (e.g., two sections), such that any unfused powder can be removed prior to the sections being fused together.

The stem 602 may extend distally from the shaft 608. During a humeral limb replacement procedure, the stem 602 is inserted into humerus while the shaft 608 abuts the humerus. The length of the stem 602 can be determined once the length of the shaft 608 is determined. For example, the overall length of the humeral limb replacement implant 600 can be determined first (e.g., based on an overall size of the patient's humerus), then the length of the shaft 608 can be determined (e.g., based on the proximal portion of the patient's humerus that needs to be replaced), and then the length of the stem 602 is determined (e.g., the remaining length based on the overall length and the length of the shaft 608). The stem 602 may have a substantially circular cross section. As another example, the stem 602 may have an oval-shaped cross section. The shape of the cross section of the stem 602 may vary along the length of the stem 602. The size of the cross section of the stem 602 may vary along the length of the stem 602. For example, the stem 602 may taper (e.g., decrease in diameter, circumference, and/or width) from a proximal end to the distal end 606. The distal end 606 can be rounded. As another example, the distal end 606 can be pointed or curved to aid in driving the humeral limb replacement implant 600 into the humerus.

The stem 602 may include stem patch 620. The stem patch 620 can be located at a proximal end of the stem 620. The stem patch 620 may facilitate attachment of muscle, ligaments, and/or other tissue. For example, the stem patch 620 can be a porous structure that facilitates tissue ingrowth and remodeling and vascularization. The porous structure can be created by a porous design and/or a porous material. The stem patch 620 can be shaped, sized, and positioned to facilitate good reconstruction by aligning with zones specific to anatomic attachment areas. The stem patch 620 may extend around the entire circumference of the stem 602. As another example, the stem patch 620 may surround a portion of the circumference of the stem 602. In another example, the stem patch 620 can be a continuous, uniform patch. In another example, the stem patch 620 can be a plurality of rings along a portion of the length of the stem 602. In another example, the stem patch 620 can be linear patches along a portion of the length of the stem 602. The stem patch 620 can be shaped as needed to align with the anatomic attachment areas. In an example, the characteristics (e.g., shape, size, and/or position) of the stem patch 620 can be customized to meet the specific needs of a patient. The stem patch 620 can be recessed in the stem 602. As another example, the stem patch 620 may have a diameter larger than the stem 602. As another example, the stem patch 620 may have a diameter that gradually increases from a distal end to a proximal end of the stem patch 620.

The shaft 608 can be free form to follow a natural shape of a humeral bone. The length of the shaft 608 of the humeral limb replacement implant 600 can be determined based on the size selected for the humeral limb replacement implant 600 and truncated to meet the needs of the patient. The cross-sectional size of the shaft 608 can be larger than the stem 602. The cross-sectional shape of the shaft 608 may change along the length of the shaft 608. For example, portions of the shaft 608 may have a substantially circular cross section, a substantially triangular cross section, and/or a substantially oval-shaped cross section. In an example, a diameter or circumference of the shaft 608 may vary along the length of the shaft 608 of the humeral limb replacement implant 600. In an example, the exterior of the shaft 608, or portions thereof, can be smoothed and/or rounded. The smooth and/or rounded contour may allow musculature to interface more easily with the humeral limb replacement implant 600 and provide an increase in function of the humeral limb replacement implant 600. The smooth and/or rounded contour of the shaft 608 may create a more pain free result for a patient.

The shaft 608 can be hollow. For example, the shaft 608 may have a wall with a thickness of approximately 1.5 mm. The shaft 608 being hollow may create a lighter humeral limb replacement implant 600. If the weight of the humeral limb replacement implant 600 is reduced, the patient can be able to bear additional weight in the arm with the humeral limb replacement implant 600. Specifically, after a humeral limb replacement implant surgery, a patient can be limited to a certain maximum amount of weight bearing. While the maximum amount of weight bearing will not change, by reducing the weight required to lift the humeral limb replacement implant, additional weight can be allocated to lifting objects.

The shaft 608 may include a shaft patch 610. The shaft patch 610 can be positioned along a portion of the shaft 608. The shaft patch 610 may facilitate attachment of muscle, ligaments, and/or other tissue. For example, the shaft patch 610 can be a porous structure that facilitates tissue ingrowth and remodeling and vascularization. The porous structure can be created by a porous design and/or a porous material. The shaft patch 610 can be shaped, sized, and positioned to facilitate good reconstruction by aligning with zones specific to anatomic attachment areas. The shaft patch 610 can be substantially oval-shaped, linear, rectangular, circular, or another shape as needed to align with the anatomic attachment areas. In an example, the characteristics (e.g., shape, size, and/or position) of the shaft patch 610 can be customized to meet the specific needs of a patient.

In an example, the shaft 608 may include a plurality of shaft patches 610. The plurality of shaft patches 610 can be substantially similar in shape and/or size. In another example, the plurality of shaft patches 610 may have different shapes and sizes. The plurality of shaft patches 610 can be positioned to align with specific anatomic attachment areas. For example, the plurality of shaft patches 610 can be positioned on a posterior side and anterior side of the shaft 608. In another example, the plurality of shaft patches 610 can be positioned on one or more of a posterior side, anterior side, medial side, and/or lateral side of the shaft 608. In another example, the plurality of shaft patches 610 can be positioned to extend around the shaft 608 in ring-like structures.

The shaft patch 610 may include shaft patch attachment holes 612. The shaft patch attachment holes 612 may receive soft suture or suture anchors. The holes 612 can be positioned within the patch 610 or can be spaced a short distance therefrom. The shaft patch 610 may include a portion of porous structure surrounded by shaft patch attachment holes 612. Additionally, or alternatively, the shaft patch 610 may include shaft patch attachment holes 612 within a portion of porous structure. The shaft patch 610 can be recessed in the shaft 608. For example, the shaft patch 610 can be recessed to allow shaft patch attachment holes 612 to receive sutures while still maintaining a substantially flush exterior of the shaft 608.

In an example, shaft patch attachment holes 612 may have attachment hole edges. The attachment hole edges can be a raised boss edge. The attachment hole edges may aid in securing the sutures. The attachment hole edges can be located on an exterior side of the shaft patch attachment holes 612. Additionally or alternatively, the attachment hole edges can be located on an interior side of the shaft patch attachment holes 612. If the shaft 608 is hollow, the attachment hole edges may aid in securing the sutures (e.g., a JuggerKnot^{®} suture or other suture or suture anchor such as one or more suture products offered by Zimmer Biomet, Inc. of Warsaw Indiana) by securing the knot of a suture. In an example, the shaft patch 610 can be recessed to allow attachment hole edges to extend from the shaft patch attachment holes 612 while maintaining a substantially flush exterior of the shaft 608. Attachment holes 612 can be chamfered or otherwise shaped to assist in guiding anchors or reducing friction on suture.

The proximal end 604 of the shaft 608 may have a substantially circular cross section. The proximal end 604 may have a greater width, diameter, or circumference than the shaft 608. The proximal end 604 may include a proximal surface 614. The proximal surface 614 can be substantially flat. The proximal surface 614 can be positioned such that a central axis of the shaft 608 and a central axis of the proximal surface 614 create an angle. The proximal surface 614 can be substantially flat to couple a proximal implant to the proximal end 604 while maintaining a substantially smooth exterior at the interface. The proximal surface 614 may include a proximal aperture 622 along the central axis of the proximal surface 614. The proximal aperture 622 may receive the proximal implant to secure the proximal implant to the proximal end 604 of the humeral limb replacement implant 600. For example, the proximal aperture 622 can include a snap configuration. The proximal implant received by the proximal aperture 622 can be a humeral tray or a humeral head.

The proximal end 604 may include a proximal patch 616. The proximal patch 616 may facilitate attachment of muscle, ligaments, and/or other tissue. For example, the proximal patch 616 can be a porous structure that facilitates tissue ingrowth and remodeling and vascularization. The porous structure can be created by a porous design and/or a porous material. The proximal patch 616 can be shaped, sized, and positioned to facilitate good reconstruction by aligning with zones specific to anatomic attachment areas. The proximal patch 616 can be substantially oval-shaped, linear, rectangular, circular, or another shape as needed to align with the anatomic attachment areas. In an example, the characteristics (e.g., shape, size, and/or position) of the proximal patch 616 can be customized to meet the specific needs of a patient.

In an example, the proximal end 604 may include a plurality of proximal patches 616. The plurality of proximal patches 616 can be substantially similar in shape and size. In another example, the plurality of proximal patches 616 may have different shapes and sizes. The plurality of proximal patches 616 can be positioned to align with specific anatomic attachment areas. For example, the plurality of proximal patches 616 can be positioned on a lateral side and anterior side of the proximal end 604. In another example, the plurality of proximal patches 616 can be positioned on one or more of a posterior side, anterior side, medial side, and/or lateral side of the proximal end 604. In another example, the plurality of proximal patches 616 can be positioned to extend around the proximal end 604 in ring-like structures.

The proximal patch 616 may include a proximal patch attachment holes 618. The proximal patch attachment holes 618 may receive soft sutures. The proximal patch 616 may include a portion of porous structure surrounded by proximal patch attachment holes 618. Additionally, or alternatively, the proximal patch 616 may include proximal patch attachment holes 618 within a portion of porous structure. In an example, proximal patch attachment holes 618 may have proximal patch attachment hole edges. The proximal patch attachment hole edges can be a raised boss edge. The proximal patch attachment hole edges may aid in securing the sutures. The proximal patch attachment hole edges can be located on an exterior side of the proximal patch attachment holes 618. Additionally, or alternatively, the proximal patch attachment hole edges can be located on an interior side of the proximal patch attachment holes 618. If the shaft 608 is hollow, the proximal patch attachment hole edges may aid in securing the sutures (e.g., a JuggerKnot^{®} suture or other suture or suture anchor such as one or more suture products offered by Zimmer Biomet, Inc. of Warsaw Indiana) by securing the knot of a suture. The proximal patch 616 can be recessed in the proximal end 604. For example, the proximal patch 616 can be recessed to allow shaft patch attachment holes 618 to receive sutures while still maintaining a substantially flush exterior of the proximal end 604. As another example, the proximal patch 616 can be recessed to allow proximal patch attachment hole edges to extend from the proximal patch attachment holes 618 while maintaining a substantially flush exterior of the proximal end 604.

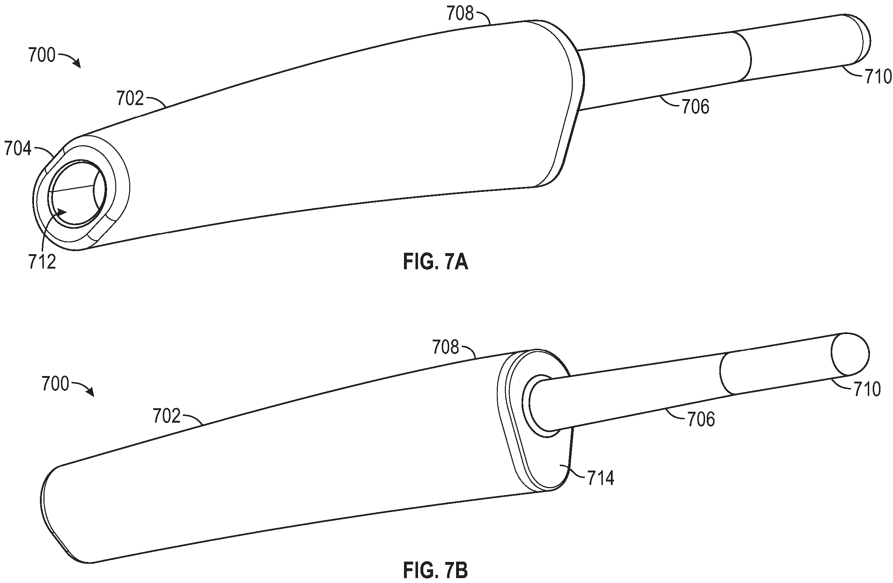

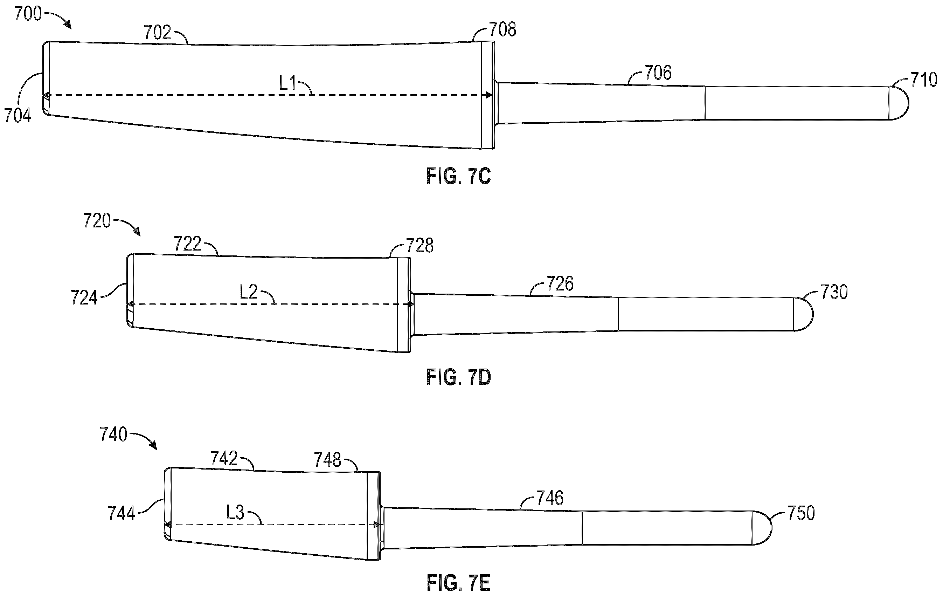

FIG. 7A-7C illustrate various views of a first partial distal humeral limb replacement implant 700. The partial humeral replacement, such as the first partial distal humeral limb replacement implant 700 can use the distal structure shape of one or more of the total implants of FIGS. 2-4 but can be truncated and have a stem to match patient's needs in preserving natural bone of the proximal humerus. The humeral limb replacement implant 700 includes a stem 706 and a shaft 702. The stem 706 includes a proximal end 710 and the shaft 702 includes a distal end 704. The humeral limb replacement implant 700 can be made using an additive metal manufacturing. For example, additive metal manufacturing may use fine, metal powders to create the humeral limb replacement implant 700. The material can be deposited layer by layer to create an object. The humeral limb replacement implant 700 can be constructed as a single piece. In another example, the humeral limb replacement implant 700 can be made in multiple sections (e.g., two sections), such that any unfused powder can be removed prior to the sections being fused together.

The stem 706 may extend proximally from the shaft 702. During a humeral limb replacement procedure, the stem 706 is inserted into humerus while the shaft 702 abuts the humerus. The length of the stem 706 can be determined once the length of the shaft 702 is determined. For example, the overall length of the humeral limb replacement implant 700 can be determined first (e.g., based on an overall size of the patient's humerus), then the shaft length L1 can be determined (e.g., based on the proximal portion of the patient's humerus that needs to be replaced), and then the length of the stem 706 is determined (e.g., the remaining length based on the overall length and the shaft length L1). The stem 706 may have a substantially circular cross section. As another example, the stem 706 may have an oval-shaped cross section. The shape of the cross section of the stem 706 may vary along the length of the stem 706. The size of the cross section of the stem 706 may vary along the length of the stem 706. For example, the stem 706 may taper (e.g., decrease in diameter, circumference, and/or width) from a distal end to the proximal end 710. The proximal end 710 can be rounded. As another example, the proximal end 710 can be pointed or curved to aid in driving the humeral limb replacement implant 700 into the humerus. In an example, the stem 706 can be revolved shape, prepared by a reamer. In another example, the stem 706 can be shaped to be a bone matching shape and prepared by a rasp.

The stem 706 may include stem patch (not shown). The stem patch can be located at a proximal end of the stem. The stem patch may facilitate attachment of muscle, ligaments, and/or other tissue. For example, the stem patch can be a porous structure that facilitates tissue ingrowth and remodeling and vascularization. The porous structure can be created by a porous design and/or a porous material. The stem patch can be shaped, sized, and positioned to facilitate good reconstruction by aligning with zones specific to anatomic attachment areas. The stem patch may extend around the entire circumference of the stem 706. As another example, the stem patch may surround a portion of the circumference of the stem 706. In another example, the stem patch can be a continuous, uniform patch. In another example, the stem patch can be a plurality of rings along a portion of the length of the stem 706. In another example, the stem patch can be linear patches along a portion of the length of the stem 706. The stem patch can be shaped as needed to align with the anatomic attachment areas. In an example, the characteristics (e.g., shape, size, and/or position) of the stem patch can be customized to meet the specific needs of a patient. The stem patch can be recessed in the stem 706. As another example, the stem patch may have a diameter larger than the stem 706. As another example, the stem patch may have a diameter that gradually increases from a proximal end to a distal end of the stem patch.

The shaft 702 can be free form to follow a natural shape of a humeral bone. The length of the shaft 702 of the humeral limb replacement implant 700 can be determined based on the size selected for the humeral limb replacement implant 700 and truncated to meet the needs of the patient. The cross-sectional size of the shaft 702 can be larger than the stem 706. The cross-sectional shape of the shaft 702 may change along the length of the shaft 702. For example, portions of the shaft 702 may have a substantially circular cross section, a substantially triangular cross section, and/or a substantially oval-shaped cross section. As another example, the shaft 702 may have an elliptical taper to assure rotational stability. In an example, a diameter or circumference of the shaft 702 may vary along the length of the shaft 702 of the humeral limb replacement implant 700. In an example, the exterior of the shaft 702, or portions thereof, can be smoothed and/or rounded. The smooth and/or rounded contour may allow musculature to interface more easily with the humeral limb replacement implant 700 and provide an increase in function of the humeral limb replacement implant 700. The smooth and/or rounded contour of the shaft 702 may create a more pain free result for a patient.

The shaft 702 can be hollow. For example, the shaft 702 may have a wall with a thickness of approximately 1.5 mm. The shaft 702 being hollow may create a lighter humeral limb replacement implant 700. If the weight of the humeral limb replacement implant 700 is reduced, the patient can be able to bear additional weight in the arm with the humeral limb replacement implant 700. Specifically, after a humeral limb replacement implant surgery, a patient can be limited to a certain maximum amount of weight bearing. While the maximum amount of weight bearing will not change, by reducing the weight required to lift the humeral limb replacement implant, additional weight can be allocated to lifting objects.

The shaft 702 may include a shaft proximal end 708. The shaft proximal end 708 may include a shaft proximal surface 714. The shaft proximal surface 714 can be substantially flat. The shaft proximal surface 714 can be substantially flat to abut an end of the natural humerus to create a flush interface.

The shaft 702 may include a shaft patch (not shown). The shaft patch can be positioned along a portion of the shaft 702. The shaft patch may facilitate attachment of muscle, ligaments, and/or other tissue. For example, the shaft patch can be a porous structure that facilitates tissue ingrowth and remodeling and vascularization. The porous structure can be created by a porous design and/or a porous material. The shaft patch can be shaped, sized, and positioned to facilitate good reconstruction by aligning with zones specific to anatomic attachment areas. The shaft patch can be substantially oval-shaped, linear, rectangular, circular, or another shape as needed to align with the anatomic attachment areas. In an example, the characteristics (e.g., shape, size, and/or position) of the shaft patch can be customized to meet the specific needs of a patient.

In an example, the shaft 702 may include a plurality of shaft patches. The plurality of shaft patches can be substantially similar in shape and size. In another example, the plurality of shaft patches may have different shapes and sizes. The plurality of shaft patches can be positioned to align with specific anatomic attachment areas. For example, the plurality of shaft patches can be positioned on a posterior side and anterior side of the shaft 702. In another example, the plurality of shaft patches can be positioned on one or more of a posterior side, anterior side, medial side, and/or lateral side of the shaft 702. In another example, the plurality of shaft patches can be positioned to extend around the shaft 702 in ring-like structures.

The shaft patch may include shaft patch attachment holes (not shown). The shaft patch attachment holes may receive soft sutures. The shaft patch may include a portion of porous structure surrounded by shaft patch attachment holes. Additionally, or alternatively, the shaft patch may include shaft patch attachment holes within a portion of porous structure. The shaft patch can be recessed in the shaft. For example, the shaft patch can be recessed to allow shaft patch attachment holes to receive sutures while still maintaining a substantially flush exterior of the shaft 702.

In an example, shaft patch attachment holes may have attachment hole edges. The attachment hole edges can be a raised boss edge. The attachment hole edges may aid in securing the sutures. The attachment hole edges can be located on an exterior side of the shaft patch attachment holes. Additionally, or alternatively, the attachment hole edges can be located on an interior side of the shaft patch attachment holes. If the shaft 702 is hollow, the attachment hole edges may aid in securing the sutures (e.g., a JuggerKnot^{®} suture or other suture or suture anchor such as one or more suture products offered by Zimmer Biomet, Inc. of Warsaw Indiana) by securing the knot of a suture. In an example, the shaft patch can be recessed to allow attachment hole edges to extend from the shaft patch attachment holes while maintaining a substantially flush exterior of the shaft 702.

The distal end 704 of the shaft 702 may have a substantially oval shaped cross section. The distal end 704 may have a greater width, diameter, or circumference than the proximal end 708 of the shaft 702. The distal end 704 may include a distal surface. The distal surface can be substantially flat. The distal surface can be positioned such that a central axis of the shaft 702 and a central axis of the distal surface are substantially parallel. The distal surface can be substantially flat to couple a proximal implant to the distal end 704 while maintaining a substantially smooth exterior at the interface. The distal end 704 may include a distal aperture 712 along the central axis of the distal surface. The distal aperture 712 may receive the distal implant to secure the distal implant to the distal end 704 of the humeral limb replacement implant 700. For example, the distal aperture 712 can include a snap configuration. The distal implant received by the distal aperture 712 can be an elbow implant.

FIG. 7D illustrates a second partial distal humeral limb replacement implant 720 having a shorter length on the distal end thereby preserving more proximal bone than the implant 700 of FIGS. 7A-7C. The partial humeral replacement, such as the second partial distal humeral limb replacement implant 720 can use the distal structure shape of the total implants illustrated and discussed previously but can be truncated with a stem to match patient's needs in preserving natural bone of the proximal humerus. The humeral limb replacement implant 720 includes a stem 726 and a shaft 722. The stem 726 includes a proximal end 730 and the shaft 702 includes a distal end 724.

The shaft 722 can be substantially similar to shaft 702. However, the shaft length L2 of shaft 722 can be shorter than the shaft length L1 of shaft 702. The distal end 724 of shaft 722 can be substantially similar to the distal end 704 of shaft 702. In another example, the distal end 724 of shaft 722 can be smaller than the distal end 704 of shaft 702. For example, distal end 724 of shaft 722 and shaft 722 can be sized proportionally to distal end 704 of shaft 702 and shaft 702. Distal end 724 may include a distal aperture. Distal aperture of distal end 724 can be sized substantially similar to distal aperture 712 of shaft 702, such that the same distal implant can be attached to the second partial distal humeral limb replacement implant 720 and the first partial distal humeral limb replacement implant 700. As another example, the distal aperture of distal end 724 maybe smaller than distal aperture 712 of shaft 702, such that a smaller distal implant can be attached to the second partial distal humeral limb replacement implant 720. The shaft 722 includes a shaft proximal end 728. The shaft proximal end 728 can be substantially similar to shaft proximal end 708. In another example, the shaft proximal end 728 can be smaller than the shaft proximal end 708. For example, proximal end 728 of shaft 722 and shaft 722 can be sized proportionally to proximal end 708 of shaft 702 and shaft 702.

The stem 726 can be substantially similar to stem 706. In another example, the stem 726 is shorter than stem 706. In another example, the stem 726 has a smaller diameter than stem 706. The proximal end 730 can be substantially similar to proximal end 710. In an example, the proximal end 730 may have a smaller size (e.g., diameter, length, etc.) than the proximal end 710. The size of the second partial distal humeral limb replacement implant 720 can be based on a smaller size of the plurality of humeral limb replacement implants 190 (see FIG. 2C) than the size of the first partial distal humeral limb replacement implant 700.

FIG. 7E illustrates a third partial distal humeral limb replacement implant 740 that is even more bone preserving than the examples of FIGS. 7A-7D. The humeral limb replacement implant 740 includes a stem 746 and a shaft 742. The stem 746 includes a proximal end 750 and the shaft 742 includes a distal end 744.

The shaft 742 can be substantially similar to shaft 702. However, the shaft length L3 of shaft 742 can be shorter than the shaft length L1 of shaft 702 and the shaft length L2 of shaft 722. The distal end 744 of shaft 722 can be substantially similar to the distal end 704 of shaft 702. In another example, the distal end 724 of shaft 722 can be smaller than the distal end 704 of shaft 702 and/or the distal end 724 of shaft 722. For example, distal end 744 of shaft 742 and shaft 742 can be sized proportionally to distal end 704 of shaft 702 and shaft 702. Distal end 744 may include a distal aperture. Distal aperture of distal end 744 can be sized substantially similar to distal aperture 712 of shaft 702, such that the same distal implant can be attached to the third partial distal humeral limb replacement implant 740 and the first partial distal humeral limb replacement implant 700. As another example, the distal aperture of distal end 744 maybe smaller than distal aperture 712 of shaft 702, such that a smaller distal implant can be attached to the third partial distal humeral limb replacement implant 740. The shaft 742 includes a shaft proximal end 748. The shaft proximal end 748 can be substantially similar to shaft proximal end 708. In another example, the shaft proximal end 748 can be smaller than the shaft proximal end 708 and/or the shaft proximal end 728. For example, shaft proximal end 728 and shaft 722 can be sized proportionally to shaft proximal end 708 and shaft 702.

The stem 746 can be substantially similar to stem 706. In another example, the stem 746 is shorter than stem 706 and/or stem 726. In another example, the stem 746 has a smaller diameter than stem 706 and/or stem 726. The proximal end 750 can be substantially similar to proximal end 710. In an example, the proximal end 750 may have a smaller size (e.g., diameter, length, etc.) than the proximal end 710 and/or the proximal end 730. The size of the third partial distal humeral limb replacement implant 740 can be based on a smaller size of the plurality of humeral limb replacement implants 190 (see FIG. 2C) than the size of the first partial distal humeral limb replacement implant 700 and/or the size of the second partial distal humeral limb replacement implant 720.

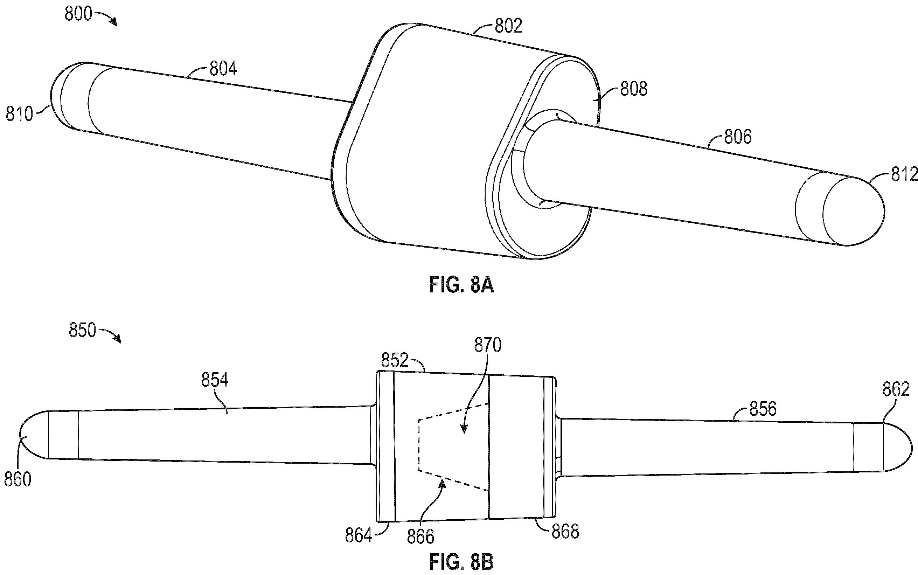

FIG. 8A illustrates a first partial intercalary humeral limb replacement implant 800. This partial humeral replacement, the first partial intercalary humeral limb replacement implant 800, can use an intercalary structure shape at a intermediate portion to preserve natural bone of the humerus on a proximal end portion and a distal end portion thereof. The partial intercalary humeral limb replacement implant 800 can be used to replace a mid-humeral shaft injury or tumor, for example. The humeral limb replacement implant 800 includes a shaft 802, a distal stem 806, and a proximal stem 804. The proximal stem 804 includes a proximal end 810 and distal stem 806 includes a distal end 812.

The humeral limb replacement implant 800 can be made using an additive metal manufacturing. For example, additive metal manufacturing may use fine, metal powders to create the humeral limb replacement implant 800. The material can be deposited layer by layer to create an object. The humeral limb replacement implant 800 can be constructed as a single piece. In another example, the humeral limb replacement implant 800 can be made in multiple sections (e.g., two sections), such that any unfused powder can be removed prior to the sections being fused together.

The shaft 802 can be free form to follow a natural shape of a humeral bone. The length of the shaft 802 of the humeral limb replacement implant 800 can be determined based on the size selected for the humeral limb replacement implant 800 and truncated on each end to meet the needs of the patient. The cross-sectional size of the shaft 802 can be larger than the distal stem 806 and the proximal stem 804. The cross-sectional shape of the shaft 802 may change along the length of the shaft 802. For example, portions of the shaft 802 may have a substantially circular cross section, a substantially triangular cross section, and/or a substantially oval-shaped cross section. In an example, a diameter or circumference of the shaft 802 may vary along the length of the shaft 802 of the humeral limb replacement implant 800. The shape of the shaft 802 can be dictated based on the location of the shaft 802 with respect to the natural humerus. In an example, the exterior of the shaft 802, or portions thereof, can be smoothed and/or rounded. The smooth and/or rounded contour may allow musculature to interface more easily with the humeral limb replacement implant 800 and provide an increase in function of the humeral limb replacement implant 800. The smooth and/or rounded contour of the shaft 802 may create a more pain free result for a patient.

The shaft 802 can be hollow. For example, the shaft 802 may have a wall with a thickness of approximately 1.5 mm. The shaft 802 being hollow may create a lighter humeral limb replacement implant 800. If the weight of the humeral limb replacement implant 800 is reduced, the patient can be able to bear additional weight in the arm with the humeral limb replacement implant 800. Specifically, after a humeral limb replacement implant surgery, a patient can be limited to a certain maximum amount of weight bearing. While the maximum amount of weight bearing will not change, by reducing the weight required to lift the humeral limb replacement implant 800, additional weight can be allocated to lifting objects.

The shaft 802 may include a shaft proximal surface 808 and a shaft distal surface. The shaft proximal surface 808 and the shaft distal surface can be substantially flat. The shaft proximal surface 808 and the shaft distal surface can be substantially flat to abut each end of the natural humerus to create a flush interface.

The shaft 802 may include a shaft patch (not shown). The shaft patch can be positioned along a portion of the shaft 802. The shaft patch may facilitate attachment of muscle, ligaments, and/or other tissue. For example, the shaft patch can be a porous structure that facilitates tissue ingrowth and remodeling and vascularization. The porous structure can be created by a porous design and/or a porous material. The shaft patch can be shaped, sized, and positioned to facilitate good reconstruction by aligning with zones specific to anatomic attachment areas. The shaft patch can be substantially oval-shaped, linear, rectangular, circular, or another shape as needed to align with the anatomic attachment areas. In an example, the characteristics (e.g., shape, size, and/or position) of the shaft patch can be customized to meet the specific needs of a patient.

In an example, the shaft 802 may include a plurality of shaft patches. The plurality of shaft patches can be substantially similar in shape and size. In another example, the plurality of shaft patches may have different shapes and sizes. The plurality of shaft patches can be positioned to align with specific anatomic attachment areas. For example, the plurality of shaft patches can be positioned on a posterior side and anterior side of the shaft 802. In another example, the plurality of shaft patches can be positioned on one or more of a posterior side, anterior side, medial side, and/or lateral side of the shaft 802. In another example, the plurality of shaft patches can be positioned to extend around the shaft 802 in ring-like structures.

The shaft patch may include shaft patch attachment holes (not shown). The shaft patch attachment holes may receive soft sutures. The shaft patch may include a portion of porous structure surrounded by shaft patch attachment holes. Additionally or alternatively, the shaft patch may include shaft patch attachment holes within a portion of porous structure. The shaft patch can be recessed in the shaft. For example, the shaft patch can be recessed to allow shaft patch attachment holes to receive sutures while still maintaining a substantially flush exterior of the shaft 802.

In an example, shaft patch attachment holes may have attachment hole edges. The attachment hole edges can be a raised boss edge. The attachment hole edges may aid in securing the sutures. The attachment hole edges can be located on an exterior side of the shaft patch attachment holes. Additionally or alternatively, the attachment hole edges can be located on an interior side of the shaft patch attachment holes. If the shaft 802 is hollow, the attachment hole edges may aid in securing the sutures (e.g., a JuggerKnot^{®} suture or other suture or suture anchor such as one or more suture products offered by Zimmer Biomet, Inc. of Warsaw Indiana) by securing the knot of a suture. In an example, the shaft patch can be recessed to allow attachment hole edges to extend from the shaft patch attachment holes while maintaining a substantially flush exterior of the shaft 802.

The proximal stem 804 may extend proximally from the shaft 802. During a humeral limb replacement procedure, the proximal stem 804 is inserted into humerus while the shaft 802 abuts a proximal portion of the humerus. The length of the proximal stem 804 can be determined once the length and position of the shaft 802 is determined. The proximal stem 804 may have a substantially circular cross section. As another example, the proximal stem 804 may have an oval-shaped cross section. The shape of the cross section of the proximal stem 804 may vary along the length of the proximal stem 804. The size of the cross section of the proximal stem 804 may vary along the length of the proximal stem 804. For example, the proximal stem 804 may taper (e.g., decrease in diameter, circumference, and/or width) from a distal end to the proximal end 810. The proximal end 810 can be rounded. As another example, the proximal end 810 can be pointed or curved to aid in driving the humeral limb replacement implant 800 into the humerus. In an example, the proximal stem 804 can be revolved shape, prepared by a reamer. In another example, the proximal stem 804 can be shaped to be a bone matching shape and prepared by a rasp.

The distal stem 806 may extend proximally from the shaft 802. During a humeral limb replacement procedure, the distal stem 806 is inserted into humerus while the shaft 802 abuts a distal portion of the humerus. The length of the distal stem 806 can be determined once the length and position of the shaft 802 is determined. The distal stem 806 may have a substantially circular cross section. As another example, the distal stem 806 may have an oval-shaped cross section. The shape of the cross section of the distal stem 806 may vary along the length of the distal stem 806. The size of the cross section of the distal stem 806 may vary along the length of the distal stem 806. For example, the distal stem 806 may taper (e.g., decrease in diameter, circumference, and/or width) from a proximal end to the distal end 812. The distal end 812 can be rounded. As another example, the distal end 812 can be pointed or curved to aid in driving the humeral limb replacement implant 800 into the humerus. In an example, the distal stem 806 can be revolved shape, prepared by a reamer. In another example, the distal stem 806 can be shaped to be a bone matching shape and prepared by a rasp.

The proximal stem 804 and the distal stem 806 can be substantially similar in length. In another example, the proximal stem 804 and the distal stem 806 may have different lengths. For example, one of the proximal stem 804 or the distal stem 806 can be longer than the other. In an example, the length of the proximal stem 804 and the distal stem 806 may correspond to a length of the remaining humerus bone.

The proximal stem 804 and/or the distal stem 806 may include a stem patch (not shown). The stem patch can be located at a distal end of the proximal stem 804. The stem patch can be located at a proximal end of the distal stem 806. The stem patch may facilitate attachment of muscle, ligaments, and/or other tissue. For example, the stem patch can be a porous structure that facilitates tissue ingrowth and remodeling and vascularization. The porous structure can be created by a porous design and/or a porous material. The stem patch can be shaped, sized, and positioned to facilitate good reconstruction by aligning with zones specific to anatomic attachment areas. The stem patch may extend around the entire circumference of the proximal stem 804 and/or the distal stem 806. As another example, the stem patch may surround a portion of the circumference of the proximal stem 804 and/or the distal stem 806. In another example, the stem patch can be a continuous, uniform patch. In another example, the stem patch can be a plurality of rings along a portion of the length of the proximal stem 804 and/or the distal stem 806. In another example, the stem patch can be linear patches along a portion of the length of the proximal stem 804 and/or the distal stem 806. The stem patch can be shaped as needed to align with the anatomic attachment areas. In an example, the characteristics (e.g., shape, size, and/or position) of the stem patch can be customized to meet the specific needs of a patient. The stem patch can be recessed in the proximal stem 804 and/or the distal stem 806. As another example, the stem patch may have a diameter larger than the proximal stem 804 and/or the distal stem 806. As another example, the stem patch may have a diameter that gradually increases toward the shaft 802.

FIG. 8B illustrates a second partial intercalary humeral limb replacement implant 850. The partial humeral replacement, such as the second partial intercalary humeral limb replacement implant 850, can use an intercalary structure shape, to preserve natural bone of the proximal portion and the distal portion of the humerus. The partial intercalary humeral limb replacement implant 850 can be used to replace a mid-humeral shaft injury or tumor.

The humeral limb replacement implant 850 can be made using an additive metal manufacturing. For example, additive metal manufacturing may use fine, metal powders to create the humeral limb replacement implant 850. The material can be deposited layer by layer to create an object. The humeral limb replacement implant 850 can be constructed as a single piece. In another example, the humeral limb replacement implant 850 can be made in multiple sections (e.g., two sections), such that any unfused powder can be removed prior to the sections being fused together.

The humeral limb replacement implant 850 includes a shaft 852, a proximal stem 854, and a distal stem 856. The proximal stem 854 includes a proximal end 860 and the distal stem 856 includes a distal end 862. The shaft 852 includes a shaft proximal portion 864 and a shaft distal portion 868.

The proximal stem 854 can be substantially similar to proximal stem 804. The proximal end 860 can be substantially similar to proximal end 810. The distal stem 856 can be substantially similar to distal stem 806. The distal end 862 can be substantially similar to distal end 812.

The shaft 852 can be free form to follow a natural shape of a humeral bone. The length of the shaft 852 of the humeral limb replacement implant 850 can be determined based on the size selected for the humeral limb replacement implant 850 and truncated on each end to meet the needs of the patient. The cross-sectional size of the shaft 852 can be larger than the distal stem 856 and the proximal stem 854. The cross-sectional shape of the shaft 852 may change along the length of the shaft 852. For example, portions of the shaft 852 may have a substantially circular cross section, a substantially triangular cross section, and/or a substantially oval-shaped cross section. In an example, a diameter or circumference of the shaft 852 may vary along the length of the shaft 852 of the humeral limb replacement implant 850. The shape of the shaft 852 can be dictated based on the location of the shaft 852 with respect to the natural humerus. In an example, the exterior of the shaft 852, or portions thereof, can be smoothed and/or rounded. The smooth and/or rounded contour may allow musculature to interface more easily with the humeral limb replacement implant 850 and provide an increase in function of the humeral limb replacement implant 850. The smooth and/or rounded contour of the shaft 852 may create a more pain free result for a patient.

In the illustrated example, the shaft 852 is divided into two pieces: a shaft proximal portion 864 and a shaft distal portion 868. The shaft proximal portion 864 includes a shaft proximal portion aperture 866. The shaft distal portion 868 includes a shaft distal portion protrusion 870. For example, the shaft distal portion protrusion 870 can be a morse taper. The shaft distal portion protrusion 870 interfaces with the shaft proximal portion aperture 866. In an example, the shaft distal portion protrusion 870 and the shaft proximal portion aperture 866 interface with a snap fit. In another example, the shaft distal portion protrusion 870 and the shaft proximal portion aperture 866 interfaces with a snug fit.

The shaft proximal portion 864 and the shaft distal portion 868 of the shaft can be installed as similar to the shaft 852. However, after installation of the second partial intercalary humeral limb replacement implant 850, the patient may grow. For example, in cases of juvenile midshaft tumors, or injuries, the shaft distal portion protrusion 870 and the shaft proximal portion aperture 866 can be interfaced. Then, the shaft distal portion protrusion 870 and the shaft proximal portion aperture 866 can later be separated to allow a segment to be installed between the shaft proximal portion 864 and the shaft distal portion 868. Adding a segment between the shaft proximal portion 864 and the shaft distal portion 868 allows the humerus to be lengthened. The lengthening of the second partial intercalary humeral limb replacement implant 850 can allow the bone with the implant to match the length of the natural bone.

In an example, the segment is sized and shaped substantially similar to the shaft 852. For example, a proximal side of the segment can be sized to align with the shaft proximal portion 864 to create a flush interface between the segment and the shaft proximal portion 864. For example, the distal side of the segment can be sized to align with the shaft distal portion 868 to create a flush interface between the segment and the shaft distal portion 868. For example, the segment has a taper to provide flush interfacing with the shaft proximal portion 864 and the shaft distal portion 868. In an example, the segment includes an aperture and a protrusion. For example, the aperture of the segment allows the shaft distal portion protrusion 870 to extend into the segment, and the protrusion of the segment extends into the shaft proximal portion aperture 866. Therefore, the segment interfaces with both the shaft proximal portion 864 and the shaft distal portion 868.

The shaft proximal portion 864 includes a shaft proximal surface and the shaft distal portion 868 includes a shaft distal surface. In example, the shaft proximal surface and the shaft distal surface are substantially flat. The shaft proximal surface and the shaft distal surface can be substantially flat to abut each end of the natural humerus to create a flush interface.

The shaft 852 may include a shaft patch (not shown). The shaft patch can be positioned along a portion of the shaft 852. The shaft patch may facilitate attachment of muscle, ligaments, and/or other tissue. For example, the shaft patch can be a porous structure that facilitates tissue ingrowth and remodeling and vascularization. The porous structure can be created by a porous design and/or a porous material. The shaft patch can be shaped, sized, and positioned to facilitate good reconstruction by aligning with zones specific to anatomic attachment areas. The shaft patch can be substantially oval-shaped, linear, rectangular, circular, or another shape as needed to align with the anatomic attachment areas. In an example, the characteristics (e.g., shape, size, and/or position) of the shaft patch can be customized to meet the specific needs of a patient.

In an example, the shaft 852 may include a plurality of shaft patches. For example, the shaft proximal portion 864 and the shaft distal portion 868 each include at least one shaft patch. The plurality of shaft patches can be substantially similar in shape and size. In another example, the plurality of shaft patches may have different shapes and sizes. The plurality of shaft patches can be positioned to align with specific anatomic attachment areas. For example, the plurality of shaft patches can be positioned on a posterior side and anterior side of the shaft 852. In another example, the plurality of shaft patches can be positioned on one or more of a posterior side, anterior side, medial side, and/or lateral side of the shaft 852. In another example, the plurality of shaft patches can be positioned to extend around the shaft 852 in ring-like structures.

The shaft patch may include shaft patch attachment holes (not shown). The shaft patch attachment holes may receive soft sutures. The shaft patch may include a portion of porous structure surrounded by shaft patch attachment holes. Additionally, or alternatively, the shaft patch may include shaft patch attachment holes within a portion of porous structure. The shaft patch can be recessed in the shaft. For example, the shaft patch can be recessed to allow shaft patch attachment holes to receive sutures while still maintaining a substantially flush exterior of the shaft 852.

In an example, shaft patch attachment holes may have attachment hole edges. The attachment hole edges can be a raised boss edge. The attachment hole edges may aid in securing the sutures. The attachment hole edges can be located on an exterior side of the shaft patch attachment holes. Additionally, or alternatively, the attachment hole edges can be located on an interior side of the shaft patch attachment holes. If the shaft 852 is hollow, the attachment hole edges may aid in securing the sutures (e.g., a JuggerKnot^{®} suture or other suture or suture anchor such as one or more suture products offered by Zimmer Biomet, Inc. of Warsaw Indiana) by securing the knot of a suture. In an example, the shaft patch can be recessed to allow attachment hole edges to extend from the shaft patch attachment holes while maintaining a substantially flush exterior of the shaft 852.

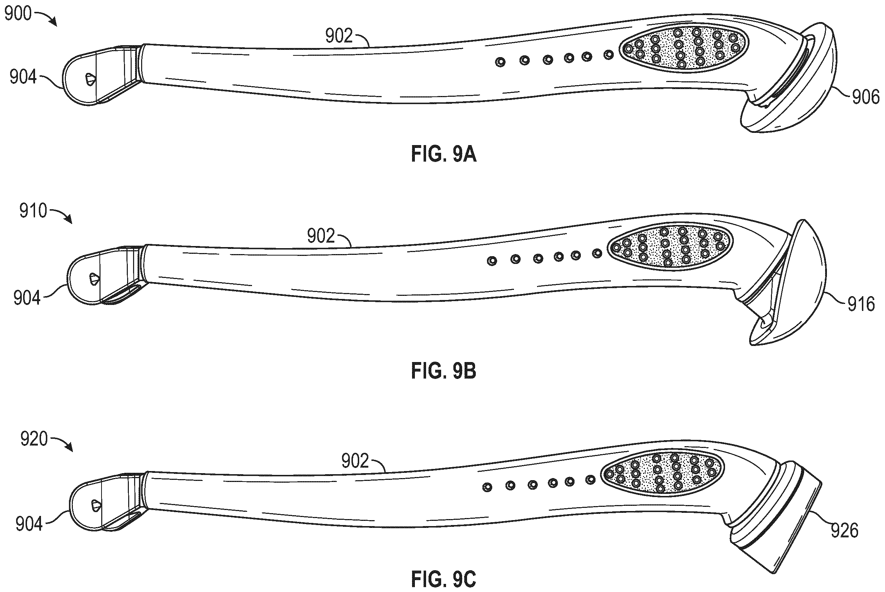

FIGS. 9A-9C illustrates three full humeral limb replacement implant assemblies 900, 910, and 920. The three full humeral limb replacement implant assemblies 900, 910, and 920 each include a shaft 902 and a distal implant component 904. The three full humeral limb replacement implant assemblies 900, 910, and 920 include a differently shaped proximal implant component. The shaft 902 can be substantially similar to the shafts described above (e.g., shaft 302).

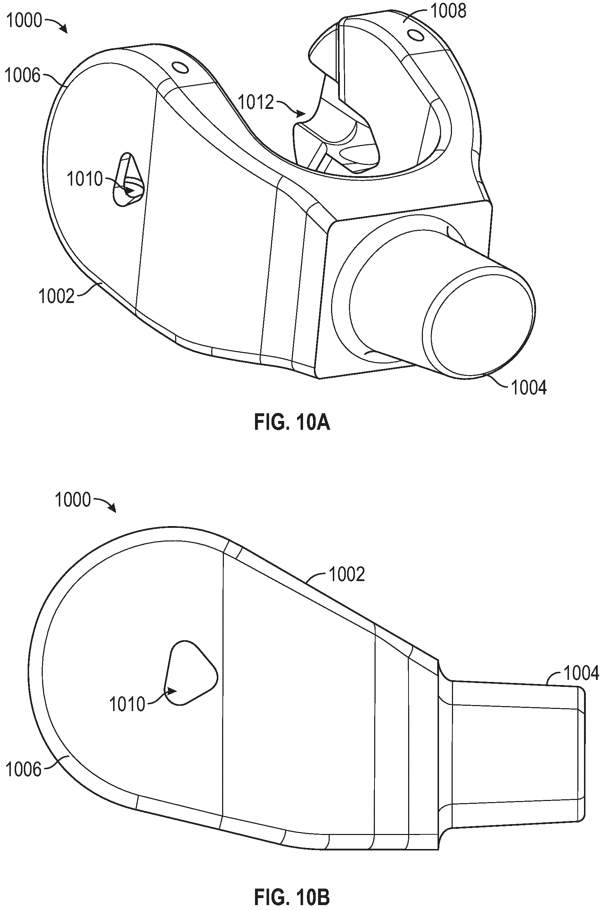

The shaft 902 accepts the distal implant component 904. The distal implant component 904 is an elbow component. In an example, the distal implant component 904 snaps into the shaft 902. In an example, the distal implant component 904 screws into the shaft 902. The distal implant component 904 is described in further detail below (see FIGS. 10A and 10B).

The shaft 902 accepts the proximal implant component. In an example, proximal implant component is a humeral head adapter proximal implant component 906. The humeral head adapter proximal implant component 906 includes a rounded surface configured to interact with a humeral head cup. The humeral head adapter proximal implant component 906 includes a circular edge.

In an example, the proximal implant component is a comprehensive proximal implant component 916. The comprehensive proximal implant component 916 includes a rounded surface configured to interact with a humeral head cup. The comprehensive proximal implant component 916 includes a comprehensive edge that includes a first edge and a second edge that extend at different angles toward the shaft 902.

In an example, the proximal implant component is a round taper reverse tray proximal implant component 926. The round taper reverse tray proximal implant component 926 includes a concave surface. The round taper reverse tray proximal implant component 926 is configured to receive an implant head.

FIGS. 10A-10B illustrate various views of a distal implant component 1000 for a humeral limb replacement implant assembly (e.g., the humeral limb replacement implant assembly of FIGS. 9A-9C). The distal implant component 1000 is an elbow component. The distal implant component 1000 includes a body 1002 and a connection protrusion 1004.

Connection protrusion 1004 is configured to couple the distal implant component 1000 to an aperture of the humeral limb replacement implant assembly. In an example, the connection protrusion 1004 has a circular cross section. In another example, the connection protrusion 1004 has an oval shaped cross section. In another example, the connection protraction 1004 has a cross section shaped to match the aperture of the humeral limb replacement implant assembly. In an example, the connection protrusion 1004 is a morse taper.

In the illustrated example, body 1002 includes a medial edge 1006 and a lateral edge 1008. The medial edge 1006 and the lateral edge 1008 engage with the natural elbow joint of the created by the ulna and radius.

As illustrated, the medial edge 1006 includes an aperture 1010. In an example, aperture 1010 provides means for ligament attachment, securement, and/or engagement. In an example, the lateral edge 1008 includes an aperture.

As illustrated, the lateral edge 1008 includes an interior groove 1012. In an example, the interior groove 1012 engages with the tibia and fibula to provide movement of the elbow joint. In an example, the interior groove 1012 extends along a portion of an interior of the lateral edge 1008. In an example, the interior groove 1012 has a first dimension at a distal location and a second, smaller, dimension at a proximal location. In an example, the medial edge 1006 include an interior groove.

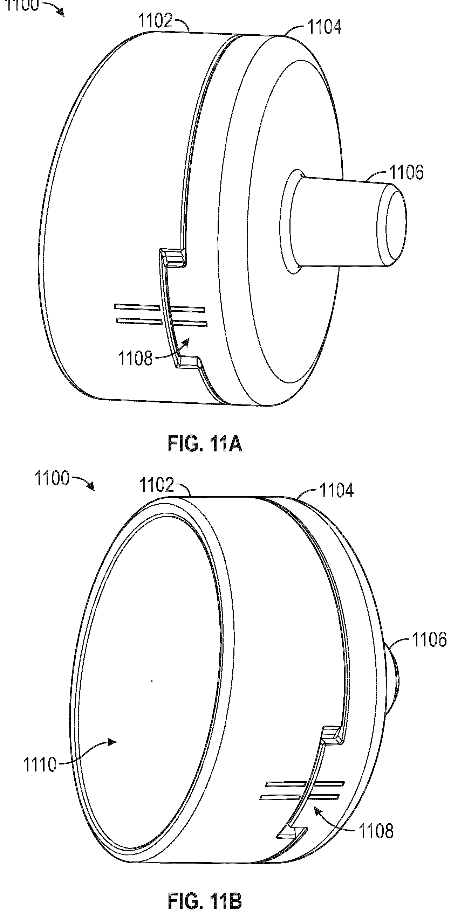

FIGS. 11A-11B illustrate various views of a proximal implant component 1100 for a humeral limb replacement implant assembly (e.g., the humeral limb replacement implant assembly of FIGS. 9A-9C). The proximal implant component 1100 can be a comprehensive baseplate or bearing assembly. In the illustrated example, the proximal implant component 1100 includes a scapular interface component 1102 and a humeral interface component 1104.

The scapular interface component 1102 is proximal the scapula for interfacing with a round ball component. The humeral interface component 1104 is proximal the humerus and couples with a humeral limb replacement implant assembly. For example, the humeral interface component 1104 includes a coupling mechanism 1106. In the illustrated, the humeral coupling mechanism 1106 is a protrusion configured engage with the humeral limb replacement implant assembly. For example, the humeral coupling mechanism 1106 may have a taper (e.g., decrease in diameter, circumference, and/or width) to couple to the humeral limb replacement implant assembly. In another example, the humeral coupling mechanism 1106 can be threaded.

The scapular interface component 1102 and the humeral interface component 1104 are removably coupled via coupling mechanism 1108. For example, the coupling mechanism 1108 is a snap fit mechanism.

The scapular interface component 1102 includes a concave interior 1110. Concave interior 1110 interfaces with a round ball component. For example, the concave interior 1110 can be sized and shaped to fit a particular ball component size. In another example, the concave interior 1110 can be sized and shaped to fit a variety of ball components, such that the same proximal implant component 1100 can be used for a variety of sizes of ball components. In another example, the scapular interface component 1102 is made via additive manufacturing, such that the concave interior 1110 is infinitely adjustable and can accommodate all round ball components.

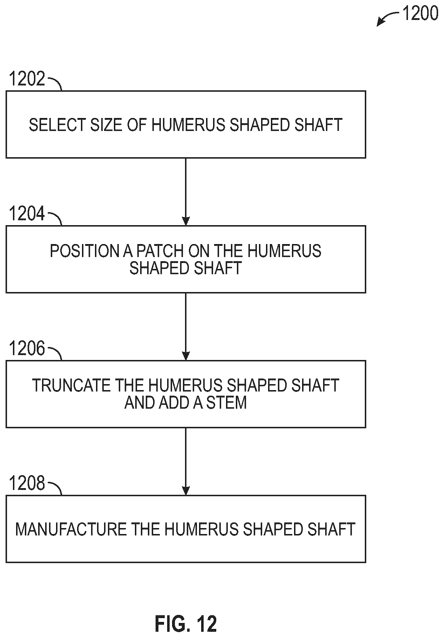

FIG. 12 illustrates an example of a method of manufacturing a limb replacement implant. At 1202, a size of a shaft of a limb replacement implant is selected. For example, the size of the shaft is selected from pre-sized implants. In an example, the pre-sized implants are the implants of FIG. 2C, which defines five sizes of implants: XS, S, M, L, and XL. These shapes are the result of a computer-aided design, anatomic library, one or more images such as x-ray, CT, MRI, etc., from which the pre-sized implants can be selected. For example, the surgeon, physician, or another medical professional selects the size of the implant based on the size of the patient. In another example, images of a patient's humerus are obtained, and the collected images are used to select the size of the implant. For example, the images of the patient's humerus are used to automatically select the pre-sized implant that is closest to the patient's humerus size. In another example, the images of the patient's humerus are used to define a custom implant with a shape and features that align with the needs of the patient.

At 1204, at least one patch is positioned on the shaft. For example, the at least one patch can be a porous structure and/or apertures. For example, the at least one patch is positioned similar to those shown in FIG. 2C. In another example, the at least one patch is positioned in a predetermined location(s) depending on the size of the pre-sized implants. As another example, the anatomical locations of all major muscle and/or ligament attachments can be defined in the system so that the at least one patch is positioned based on the locations of all major muscle and/or ligament attachments. For example, the images of the patient's humerus are used to determine the location of all major muscle and/or ligament attachments, or the images of the patient's humerus are used to determine where on the humerus the predetermined locations align.

At 1206, the shaft is optionally truncated, and a stem is added to the limb replacement implant. For example, the shaft is truncated to create a proximal humeral implant, a distal humeral implant, or an intercalary implant. In an example, the shaft is truncated using predetermined lengths, as shown in FIG. 4. Such predetermined truncation lengths can be stored in the system as standard parts for a shaft. In another example, the shaft is truncated as a unique length. For example, the length of the patient's humerus, or a portion that needs replacement, is used to determine the length of the shaft after truncation. As another example, the images of the patient's humerus are used to determine the location of truncation. Once the shaft is truncated, a stem is added to allow the shaft to be attached to the natural humerus. More specifically, the stem is added to allow the stem to be inserted into the remaining natural humerus bone to secure the shaft into position. In an example, when the stem is added, a diameter of the stem is sized to fit within the diameter of the natural humerus. In an example, when the stem is added, a length of the stem is sized to fit within a remaining length of the natural humerus without any protrusion.

In an example, the proximal humeral implant, the distal humeral implant, or the intercalary implant are substantially similar to those shown in FIGS. 5A - 8B.

At 1208, the shaft is manufactured using additive manufacturing techniques. In an example, the additive manufacturing techniques create porous structures of the implant. In an example, different materials are used when to create porous structures of the implant. For example, the limb replacement implant is made of metal.

The steps or operations of the method 1200 are illustrated in a particular order for convenience and clarity; many of the discussed operations can be performed in a different sequence or in parallel without materially impacting other operations. The method 1200 as discussed includes operations performed by multiple different actors, devices, and/or systems. It is understood that subsets of the operations discussed in the method 1200 can be attributable to a single actor, device, or system could be considered a separate standalone process or method.

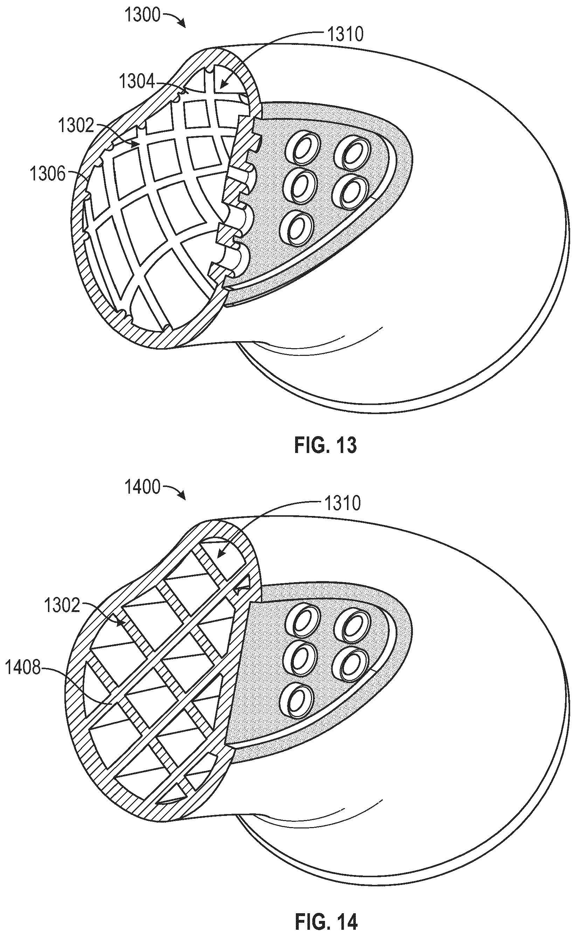

FIG. 13 illustrates a limb replacement implant 1300 according to another example. FIG. 13 shows a cross-section of the limb replacement implant 1300 having internal reinforcing structures 1302. Such internal reinforcing structures 1302 can be a lattice of raised ribs 1304 on some or all internal surfaces 1306, for example. The raise ribs 1304 can be raised between 1 mm and 2 mm from a remainder of the internal surfaces 1306, for example. The raise ribs 1304 can extend along a internal space 1310 of the limb replacement implant 1300.

FIG. 14 illustrates a limb replacement implant 1400 having internal reinforcing structures 1302 that are a matrix of honeycomb 1408 filling all or part of an entire internal space 1310 formed by the shaft of the limb replacement implant 1400. Additionally contemplated examples can include a combination of honeycomb and raised ribs of FIGS. 13 and 14. Such combination can depend upon loads the limb replacement implant 1300, 1400 will carry, for example. Heavier load bearing bones, such as the femur could use honeycomb 1408, while bones such as the humerus subject to lighter loading could utilize the raised ribs 1304. In other examples, the combination can be based upon loading in a particular area or region of a single bone. For example, the raised ribs 1304 can be used in lower-loaded regions and honeycomb 1408 can be used in higher-loaded regions. Additive manufacturing can allow for creation of as many load-bearing features inside the implant as may be required to give the best combination of strength and light weight for each discrete size and potentially for each individual patient. Unfused metal from the additive manufacturing process can drain out of the internal space 1310 due to the spaced apart structures creating voids between elements of the internal reinforcing structures 1302 or can be otherwise removed so the finished implant 1300, 1400 does not have unfused powder/material trapped inside it.

The following, non-limiting examples, detail certain aspects of the present subject matter to solve the challenges and provide the benefits discussed herein, among others.

Example 1 is a limb replacement implant optionally comprising: a shaft configured to simulate an anatomical shape of a natural humerus, the shaft extending from a proximal end to a distal end; and a patch positioned on a portion of the shaft, wherein the patch is at least one of: positioned, sized or shaped as derived from an anatomical counterpart attachment from the natural humerus.

In Example 2, the subject matter of Example 1 optionally includes, wherein shaft is formed to be configured to simulate the anatomical shape of the natural humerus by additive manufacturing.

In Example 3, the subject matter of Examples 1-2 optionally includes, wherein the patch comprises a porous structure or material and is configured to facilitate anatomic attachment to a soft tissue associated with the natural humerus.

In Example 4, the subject matter of Example 3 optionally includes, wherein the patch further comprises at least one aperture configured to receive a suture.

In Example 5, the subject matter of Example 4 optionally includes, wherein the at least one aperture comprises a raised edge.

In Example 6, the subject matter of Examples 1-5 optionally includes, wherein the patch is positioned in an anatomical attachment location of soft tissue associated with the natural humerus.

In Example 7, the subject matter of Examples 1-6 optionally includes, wherein at least a portion of the shaft includes a cross-sectional shape of a circle or an oval, and wherein the cross-sectional shape of the shaft changes from the proximal end to the distal end.

In Example 8, the subject matter of Example 7 optionally includes, wherein the cross-sectional shape of a portion of the distal end is oval shaped and the cross-sectional shape of a portion of the proximal end is circle shaped.

In Example 9, the subject matter of Examples 1-8 optionally includes, wherein the shaft is at least one of: hollow, elongate and having at least one curvature proximal-distal, elongate with the proximal portion configured to simulate at least a portion of a head of the humerus, elongate and having two or more curvatures along a lateral side surface thereof, elongate and having two or more curvatures along a medial side surface thereof.

In Example 10, the subject matter of Examples 1-9 optionally includes, wherein the shaft comprises a smooth rounded exterior save for the patch.

In Example 11, the subject matter of Examples 1-10 optionally includes, wherein the proximal end of the shaft defines at least a portion of a humeral head that is configured to articulate with a natural or prosthetic glenoid.

In Example 12, the subject matter of Examples 1-11 optionally includes, wherein the distal end of the shaft forms a portion of an elbow joint.

In Example 13, the subject matter of Examples 1-12 optionally includes, wherein the shaft is truncated and further comprises a stem extending from the shaft.

Example 14 is a limb replacement implant optionally comprising: an elongate shaft having a proximal end and a distal end; a proximal stem extending from the proximal end of the elongate shaft; and a distal stem extending from the distal end of the elongate shaft, wherein the elongate shaft, the proximal stem, and the distal stem are formed by additive manufacturing.

In Example 15, the subject matter of Example 14 optionally includes, wherein at least a portion of the elongate shaft, the proximal stem, and/or the distal stem is hollow.

In Example 16, the subject matter of Examples 14-15 optionally includes, wherein the elongate shaft comprises a shaft proximal portion and a shaft distal portion, and wherein the shaft proximal portion and the shaft distal portion are removably coupled.

In Example 17, the subject matter of Example 16 optionally includes, a shaft segment, wherein the shaft segment is configured to be removably coupled between the shaft proximal portion and the shaft distal portion to lengthen the limb replacement implant.

In Example 18, the subject matter of Examples 14-17 optionally includes, at least one patch positioned in an anatomical attachment location of soft tissue associated with a natural humerus.

Example 19 is an implant optionally comprising: an elongated shaft including hollow body portion, the elongate shaft configured to simulate a shape of at least a portion of a bone to be replaced; an attachment patch positioned along the hollow body and configured to facilitate attachment of soft tissues to the elongated shaft; a proximal end including at least one coupling feature for attachment; and a distal end including at least one coupling feature for attachment.

Example 20 is a method of manufacturing a limb replacement implant, optionally comprising: determining a size of a shaft based upon one or an anatomic library or a pre-operative scan; determining a location for a patch on the shaft; and creating the shaft using an additive manufacturing process.

In Example 21, the subject matter of Example 20 optionally includes, wherein determining the size of the shaft comprises selecting a pre-sized shaft from a plurality of pre-sized shafts or generating a custom size based on an image of a humerus of a patient.

In Example 22, the subject matter of Examples 20-21 optionally includes, truncating a portion of the shaft; and adding a stem to a truncated side of the shaft.

Example 23 is a method for generating a limb replacement implant, the method optionally comprising: determining a size of a shaft for the limb replacement implant; generating an attachment patch for the shaft; and outputting an implant design file for use in manufacturing the limb replacement implant, wherein the implant design file include, details on the shaft and the attachment patch.

In Example 24, the subject matter of Example 23 optionally includes, wherein the generating the attachment patch includes generating a plurality of attachment holes.

In Example 25, the subject matter of Examples 23-24 optionally includes, wherein the generating the attachment patch includes generating a porous surface area on the shaft.

In Example 26, the subject matter of Examples 23-25 optionally includes, wherein the generating the attachment patch for the shaft includes correlating at least one of: a position, a size or a shape of the patch from an anatomical counterpart attachment from a natural humerus.

In Example 27, the subject matter of Examples 1-26 optionally further includes, internal reinforcing structures within an internal space formed by the shaft.

In Example 28, the subject matter of Example 27 optionally includes, wherein the internal reinforcing structures comprise one of or a combination of ribs and honeycomb.

In Example 29, the subject matter of Examples 20-28 optionally includes, forming internal reinforcing structures within an internal space formed by the shaft with the additive manufacturing process.

Example 30 is at least one machine-readable medium including instructions that, when executed by processing circuitry, cause the processing circuitry to perform operations to implement of any of Examples 1-29.

Example 31 is an apparatus comprising means to implement of any of Examples 1-29.

Example 32 is a system to implement of any of Examples 1-29.

Example 33 is a method to implement of any of Examples 1-29.

Example 34, is the limb replacement implant, machine-readable medium, system or method of any one or any combination of Examples 1 - 33 can optionally be configured such that all elements or options recited are available to use or select from.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features can be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A limb replacement implant comprising:
a shaft configured to simulate an anatomical shape of a natural humerus, the shaft extending from a proximal end to a distal end; and
a patch positioned on a portion of the shaft, wherein the patch is at least one of: positioned, sized or shaped as derived from an anatomical counterpart attachment from the natural humerus.

2. The limb replacement implant of claim 1, wherein shaft is formed to be configured to simulate the anatomical shape of the natural humerus by additive manufacturing.

3. The limb replacement implant of any one of claims 1-2, wherein the patch comprises a porous structure or material and is configured to facilitate anatomic attachment to a soft tissue associated with the natural humerus.

4. The limb replacement implant of claim 3, wherein the patch further comprises at least one aperture configured to receive a suture.

5. The limb replacement implant of claim 4, wherein the at least one aperture comprises a raised edge.

6. The limb replacement implant of any one of claims 1-5, wherein the patch is positioned in an anatomical attachment location of soft tissue associated with the natural humerus.

7. The limb replacement implant of any one of claims 1-6, wherein at least a portion of the shaft includes a cross-sectional shape of a circle or an oval, and wherein the cross-sectional shape of the shaft changes from the proximal end to the distal end.

8. The limb replacement implant of claim 7, wherein the cross-sectional shape of a portion of the distal end is oval shaped and the cross-sectional shape of a portion of the proximal end is circle shaped.

9. The limb replacement implant of any one of claims 1-8, wherein the shaft is at least one of: hollow, elongate and having at least one curvature proximal-distal, elongate with the proximal portion configured to simulate at least a portion of a head of the humerus, elongate and having two or more curvatures along a lateral side surface thereof, elongate and having two or more curvatures along a medial side surface thereof.

10. The limb replacement implant of any one of claims 1-9, wherein the shaft includes internal reinforcing structures within an internal space formed by the shaft.

11. The limb replacement implant of any one of claims 1-10, wherein the proximal end of the shaft defines at least a portion of a humeral head that is configured to articulate with a natural or prosthetic glenoid.

12. The limb replacement implant of any one of claims 1-11, wherein the distal end of the shaft forms a portion of an elbow joint.

13. The limb replacement implant of any one of claims 1-12, wherein the shaft is truncated and further comprises a stem extending from the shaft.

14. A method for generating a limb replacement implant, the method comprising:
determining a size of a shaft for the limb replacement implant;
generating an attachment patch for the shaft includes correlating at least one of: a position, a size or a shape of the patch from an anatomical counterpart attachment from a natural humerus; and
outputting an implant design file for use in manufacturing the limb replacement implant, wherein the implant design file include details on the shaft and the attachment patch.

15. The method of claim 14, wherein the generating the attachment patch includes: generating a plurality of attachment holes; and
generating a porous surface area on the shaft.
